# EUROPÄISCHE PATENTSCHRIFT

(11) **EP 3 629 888 B1**
(45) Veröffentlichungstag und Bekanntmachung des Hinweises auf die Patenterteilung: **31.05.2023**
(21) Anmeldenummer: 18728121.7
(22) Anmeldetag: 29.05.2018
(51) Int. Cl.: A61B 5/00

(54) **VERFAHREN UND VORRICHTUNG ZUM BEREITSTELLEN EINES HAARBEHANDLUNGSMITTELS**
METHOD AND DEVICE FOR PROVIDING A HAIR TREATMENT AGENT
PROCÉDÉ ET DISPOSITIF POUR FOURNIR UN PRODUIT DE SOIN CAPILLAIRE

(30) Priorität: 31.05.2017 DE 102017209227
(43) Veröffentlichungstag der Anmeldung: 08.04.2020
(73) Patentinhaber: Henkel AG & Co. KGaA, 40589 Düsseldorf (DE)
(72) Erfinder: KUMPAN-BAHRAMI, Esther, 40547 Düsseldorf (DE); KATZAROV, Jordan, 40591 Düsseldorf (DE); KNUEBEL, Hans Georg, 40219 Düsseldorf (DE)
(86) Internationale Anmeldenummer: PCT/EP2018/063988
(87) Internationale Veröffentlichungsnummer: WO 2018/219895

(56) Entgegenhaltungen:
- WO-A1-01/23850
- WO-A1-2017/032636
- WO-A2-02/24071
- US-A1- 2010 139 682
- US-A1- 2015 342 515

## Beschreibung

Die Erfindung betrifft ein Verfahren und Vorrichtung zum Bereitstellen eines Haarbehandlungsmittels.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haare abgestimmt sind, und somit eine besonders hohe Wirksamkeit ermöglichen.

Bei einer Behandlung von Haar mit kosmetischen Produkten kann eine Wirkung des Produkts, z.B. eine Intensität einer Färbung, eine Wirksamkeit eines Pflegeprodukts oder eine Haarumformungswirkung einer Dauerwelle, stark von einem Schädigungsgrad des Haars abhängen.

Deshalb kann eine Ermittlung einer Schädigung des Haars von großer Bedeutung sein.

Manchen, insbesondere jungen, Friseuren kann es an Erfahrung fehlen, wie mit geschädigtem Haar umzugehen ist, z.B. welche Pflegemittel geeignet sind und/oder wie ein Haarfärbemittel (auch als Haarcoloration bezeichnet) anzumischen ist, um bei geschädigtem Haar trotzdem zu dem vom Nutzer gewünschten Färbeergebnis zu gelangen.

Die Schädigung des Haars kann durch natürliche oder künstlich herbeigeführte Vorgänge passieren. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O₂) auf das Haar aufweisen.

Die künstlich herbeigeführten Vorgänge können dabei beispielsweise ein Anwenden von Haarfärbemitteln (auch als Colorationen bezeichnet, wozu hierin auch ein Blondieren gezählt wird), und/oder ein Stylen bzw. Umformen des Haars (z.B. ein Erzeugen einer Dauerwelle) aufweisen.

Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Beim geschädigten Haar kann beispielsweise ein Cysteinsäuregehalt erhöht sein wegen einer Oxidation der im Haar sehr häufig vorkommenden Aminosäuren Cystin und Cystein zu Cysteinsäure.

Neben Cystin und Cystein können auch andere, der im menschlichen Haar vorkommenden Aminosäuren oxidiert werden.

Die Oxidation des Cystins/Cysteins zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenbeschaffenheit, z.B. eine Oberflächenrauhigkeit, negativ beeinflusst werden. Geschädigtes Haar kann beispielsweise eine höhere Oberflächenrauhigkeit aufweisen als ungeschädigtes Haar.

Das menschliche Haar besteht aus Proteinen, Lipiden, Wasser, Spurenelementen und Pigmenten. Das Faserprotein Keratin ist der Hauptbestandteil der Haare. Die Lipide, die im menschlichen Haar vorhanden sind, sind entweder frei oder kovalent gebunden. Melanine sind rötliche, braune oder schwarze Pigmente, die die Färbung der Haare bewirken.

Ergebnisse kosmetischer Behandlungen können von weiteren Eigenschaften des behandelten Haars abhängen, beispielsweise (insbesondere bei einer Coloration) von einer Haarfarbe, von einer Haarstruktur (insbesondere bei einem Styling, z.B. einer Dauerwelle, einer Glättung usw.), von einem Feuchtigkeitsgehalt (bei einem Pflegeprodukt), usw.

Deshalb kann auch eine Ermittlung der Zusammensetzung des Haars in Bezug auf die enthaltenen Aminosäuren, Lipide und Melanine sowie des Wassergehaltes von großer Bedeutung sein.

Herkömmliche, im Handel erhältliche Haarbehandlungsprodukte liegen als begrenzte Produktpalette für einen vorgegebenen Anwendungszweck vor. Beispielsweise stellt jeder Hersteller von Haarfarben eine begrenzte Palette an Haarfärbemitteln bereit. Auch wenn ein Haarschädigungsgrad des Nutzers vor dem Färben bekannt sein kann (ggf. zusätzlich zu seiner Ausgangshaarfarbe), z.B. aus Labormessungen, kann deshalb unmöglich sein, ein Wunschergebnis zu erzielen, sondern üblicherweise kann höchstens ein Ergebnis nahe am Wunschergebnis erzielt werden.

Außerdem kann es unpraktisch sein, den Haarschädigungsgrad und ggf. weitere Haarzustandsparameter wie dem Haarzustand (z.B. Haarfarbe, Haarstruktur usw.) anhand von Messungen, welche beispielsweise durch verschiedene Labors und/oder verschiedene Vorrichtungen vorgenommen werden können, zusammenzutragen, um ein Haarbehandlungsprodukt zu ermitteln.

Dokument WO 01/23850 A1 beschreibt eine Vorrichtung, die so konfiguriert ist, dass ein Körperteil gescannt wird, um dessen Farbe zu bestimmen und die Herstellung einer geeigneten kosmetischen Zusammensetzung an einer Mischeinheit der Vorrichtung auszulösen. Dokument US 2010/0139682 A1 beschreibt eine Kämmvorrichtung, die eine Lichtquelle und einen Detektor umfasst, um die Eigenschaften des Haares abzutasten und auf der Grundlage der abgetasteten Eigenschaften Verbindungen auf das Haar aufzutragen.

Die vorliegende Erfindung bezieht sich auf eine Vorrichtung zum Bereitstellen eines Haarbehandlungsmittels, aufweisend: eine tragbare Sensorvorrichtung mit mindestens einem Sensor zum Erfassen mindestens eines Sensorwerts an Haaren eines Nutzers; eine Datenverarbeitungsvorrichtung zum Ermitteln eines Haarzustands des Nutzers mittels des erfassten mindestens einen Sensorwerts, und zum computergestützten Ermitteln eines nutzerspezifischen Haarbehandlungsmittels unter Einbeziehung des ermittelten Haarzustands; und eine Haarbehandlungsmittel-Mischvorrichtung zum Anfertigen des ermittelten nutzerspezifischen Haarbehandlungsmittels, wobei der mindestens eine Sensor mindestens einen optischen Sensor zum Ermitteln eines Gehalts eines Haarinhaltsstoffes des Haars des Nutzers aufweist, wobei der Gehalt eines Haarinhaltsstoffes wenigstens ein Cysteinsäuregehalt ist, und das Ermitteln des Haarzustands zumindest ein Ermitteln eines Schädigungsgrads des Haars aufweist.

Der Haarzustand kann eine Haarschädigung und/oder einen Haarstatus aufweisen.

Die Haarschädigung kann in verschiedenen Ausführungsbeispielen eine oxidative Haarschädigung oder eine mechanische Haarschädigung sein.

Der Haarstatus kann insbesondere eine Haarfarbe, einen Gehalt von einem Haarinhaltsstoff, eine Haardicke, eine Lockigkeit von Haaren und/oder einen Grauanteil von Haaren aufweisen.

Der Haarinhaltsstoff kann insbesondere Wasser, Melanine, Lipide, Aminosäuren und Mischungen daraus umfassen. Ein bevorzugter Haarinhaltsstoff, dessen Gehalt zur Ermittlung eines Haarstatus bestimmt wird, ist Wasser.

Menschliche Haare enthalten neben den 20 kanonischen Aminosäuren Glycin, Alanin, Valin, Isoleucin, Leucin, Phenylalanin, Tyrosin, Lysin, Arginin, Histidin, Asparaginsäure, Glutaminsäure, Threonin, Serin, Glutamin, Asparagin, Methionin, Cystein, Prolin und Trypthophan ferner die Aminosäuren Cystin, Ornithin und Citrullin. Entsprechend ist es bevorzugt, dass der Haarinhaltsstoff, dessen Gehalt bestimmt wird, eine Aminosäure ausgewählt aus der Gruppe bestehend aus Glycin, Alanin, Valin, Isoleucin, Leucin, Phenylalanin, Tyrosin, Lysin, Arginin, Histidin, Asparaginsäure, Glutaminsäure, Threonin, Serin, Glutamin, Asparagin, Methionin, Cystein, Prolin, Trypthophan, Cystin, Ornithin, Citrullin und Mischungen davon ist.

Zum Ermitteln des Haarzustands des Nutzers kann das System in verschiedenen Ausführungsbeispielen eine Sensorvorrichtung aufweisen. Die Sensorvorrichtung kann mindestens einen Sensor aufweisen, beispielsweise eine Mehrzahl von Sensoren.

Hierin kann Bezug genommen werden auf "die Sensoren", beispielsweise hinsichtlich einer Datenübertragung zwischen den Sensoren und der Datenverarbeitungsvorrichtung, einer Anordnung der Sensoren, usw. Dies ist so zu verstehen, dass die Sensoren eine Gesamtheit von in der Sensorvorrichtung angeordneten Sensoren und/oder Sensorschaltkreisen aufweisen können, z.B. eine Gesamtheit von Mikrofon/en, optischem/n Sensor/en (z.B. Spektrometer, Kamera, Mikroskop), Geschwindigkeits-Sensorschaltkreis, usw., oder, sofern dies aus dem Kontext hervorgeht, einen Teil der genannten Sensoren und/oder Sensorschaltkreise.

Zum Ermitteln des Haarzustands und zum Ermitteln des Haarbehandlungsprodukts weist das System in verschiedenen Ausführungsbeispielen eine Datenverarbeitungsvorrichtung auf.

In verschiedenen Ausführungsbeispielen kann ein "ganzheitliches Ökosystem" aus intelligenten/smarten Geräten bereitgestellt werden, welches alle Funktionen von einer Problemidentifikation, d.h. einer Analyse des Haars (mittels der Sensorvorrichtung) über eine Beratung, d.h. eine Auswertung der erfassten Sensorwerte, bis hin zu einer Bereitstellung eines gewünschten Nutzens, z.B. eines Haarbehandlungsprodukts, vereint.

In verschiedenen Ausführungsbeispielen kann eine Haarbehandlungsmittel-Mischvorrichtung zum Anfertigen des ermittelten nutzerspezifischen Haarbehandlungsmittels derart mit einer (tragbaren) Sensorvorrichtung und einer Datenverarbeitungsvorrichtung zu einem System gekoppelt sein, dass ein Erfassen eines Haarzustands des Nutzers und ein Ermitteln einer auf den Haarzustand des Nutzers abgestimmten und von der Mischvorrichtung anzufertigenden Zusammensetzung des Haarbehandlungsmittels direkt vor Ort vorgenommen werden kann.

In verschiedenen Ausführungsbeispielen kann das System drei Elemente aufweisen, nämlich eine Analysevorrichtung für den Haarstatus, z.B. einen Kamm, eine Bürste oder einen auf andere Weise geformten Körper der Sensorvorrichtung, welche/r ein Mikroskop und/oder einen sonstigen Sensor aufweisen kann, eine Anzeigevorrichtung, z.B. eine Anzeige- und Eingabevorrichtung (z.B. ein Smartphone, Tablet, Laptop oder ein so genannter "Smart Mirror"), auf welcher eine App oder sonstige geeignete Software installiert sein kann, und eine Mischmaschine für ein individualisiertes Haarbehandlungsmittel, z.B. ein Haarpflegemittel und/oder ein Haarfärbemittel. Die Anzeige- und Eingabevorrichtung kann dabei einen (z.B. berührungsempfindlichen) Bildschirm aufweisen. Ferner kann die Anzeige- und Eingabevorrichtung zu einem Ermitteln einer Zusammensetzung des individualisierten Haarbehandlungsmittels eingerichtet sein. Der Smart Mirror kann eine Vorrichtung sein, welche eine Funktion eines Spiegels mit einer Funktion eines (z.B. berührungsempfindlichen) Bildschirms kombiniert, d.h. zusätzlich zu einem Spiegeln und/oder Abstrahlen von Licht (z.B. einer Spiegelung oder Darstellung des Nutzers) Informationen bereitstellen und/oder empfangen und ggf. auswerten kann. In verschiedenen Ausführungsbeispielen kann der Smart Mirror so gestaltet sein, dass er anstelle eines gespiegelten Bilds des Nutzers eine Darstellung bereitstellt, die wie ein gespiegeltes Bild des Nutzers wirkt, oder eine Darstellung des Nutzers, in welchem seine Haare verändert dargestellt sind, z.B. entsprechend eines ermittelten Behandlungsergebnisses, z.B. mit einer als Ergebnis einer Haarfärbung ermittelten Haarfarbe. Dabei kann die Darstellung derart gebildet sein, dass sie wie ein gespiegeltes Bild des Nutzers mit der neuen Haarfarbe wirkt. Die Darstellung kann beispielsweise dynamisch sein. Der Smart Mirror kann, abgesehen von hierin beschriebenen Unterschieden, auf im Wesentlichen bekannte Weise gebildet sein.

In verschiedenen Ausführungsbeispielen kann die Anzeige- und Eingabevorrichtung, z.B. der Smart Mirror, eingerichtet sein, die Mischmaschine zu steuern. Die Anzeige- und Eingabevorrichtung kann beispielsweise einen Vorschlag für eine Zusammensetzung eines Haarbehandlungsmittels (z.B. eines Haarpflege- und/oder Haarfärbemittels) bereitstellen, welchen der Nutzer (oder z.B. der Friseur, Verkäufer o.ä.) annehmen oder abändern kann. Die letztendlich angenommene Zusammensetzung kann in verschiedenen Ausführungsbeispielen an die Mischmaschine zum Herstellen des Haarbehandlungsmittels übermittelt werden.

In verschiedenen Ausführungsbeispielen kann die Eingabevorrichtung genutzt werden, um ein Wunschergebnis einzugeben, beispielsweise eine Wunschhaarfarbe, einen Wunsch-Pflegezustand, ein gewünschtes Styling (z.B. Locken mit einer abgestuften Lockigkeit oder ähnliches).

In verschiedenen Ausführungsbeispielen kann die Vorrichtung zum Bereitstellen des Haarbehandlungsmittels beim Ermitteln des Haarbehandlungsmittels das Wunschergebnis berücksichtigen, derart, dass das ermittelte und daraufhin von der Haarbehandlungsmittel-Mischvorrichtung bereitgestellte Haarbehandlungsmittel geeignet ist, am Haar des Nutzers das Wunschergebnis herbeizuführen.

In verschiedenen Ausführungsbeispielen kann die Anzeige- und Eingabevorrichtung mit der Sensorvorrichtung eine integrierte Einheit bilden, beispielsweise beim Verwenden einer Kamera und/oder eines Mikrofons eines Smartphones als Sensor, oder beispielsweise indem ein Nahinfrarotspektrometer mit einer Datenverarbeitungsvorrichtung und einem z.B. berührungsempfindlichen Display ausgerüstet ist. In verschiedenen Ausführungsbeispielen ist die Sensorvorrichtung Bestandteil eines smarten Endgeräts, vorzugsweise eines Smartphones oder eines Tablets.

In verschiedenen Ausführungsbeispielen kann die Haarbehandlungsmittel-Mischvorrichtung mit der Anzeige- und Eingabevorrichtung eine integrierte Einheit bilden, beispielsweise indem die Haarbehandlungsmittel-Mischvorrichtung mit der Datenverarbeitungsvorrichtung und der Anzeige- und Eingabevorrichtung in Form eines z.B. berührungsempfindlichen Bildschirms ausgerüstet ist. Die Anzeige- und Eingabevorrichtung kann in dem Fall eingerichtet sein, zum Initiieren einer Messung mittels der mit der Haarbehandlungsmittel-Mischvorrichtung gekoppelten Sensorvorrichtung genutzt zu werden.

In verschiedenen Ausführungsbeispielen kann mindestens eine von der Sensorvorrichtung, der Datenverarbeitungsvorrichtung und der Haarbehandlungsmittel-Mischvorrichtung zusätzlich zur Anzeigevorrichtung oder alternativ dazu eine akustische Ausgabevorrichtung aufweisen, z.B. einen Lautsprecher.

In verschiedenen Ausführungsbeispielen kann mindestens eine von der Sensorvorrichtung, der Datenverarbeitungsvorrichtung und der Haarbehandlungsmittel-Mischvorrichtung zusätzlich zum berührungsempfindlichen Bildschirm als Eingabevorrichtung oder alternativ dazu eine akustische Eingabevorrichtung aufweisen, z.B. ein Mikrofon.

In einem Fall, dass die Sensorvorrichtung das Mikrofon aufweist, kann das Mikrofon in verschiedenen Ausführungsbeispielen auch als Eingabemikrofon genutzt werden.

In verschiedenen Ausführungsbeispielen kann als Eingabevorrichtung alternativ oder zusätzlich eine andere herkömmliche Eingabevorrichtung bereitgestellt sein, z.B. eine Tastatur, eine Maus, etc.

In verschiedenen Ausführungsbeispielen kann in der Mischvorrichtung eine Mehrzahl von Einzelkomponenten bereitgestellt sein, welche anhand der ermittelten Zusammensetzung gemischt werden können, um das nutzerspezifische Haarbehandlungsmittel zu erzeugen. Die Mehrzahl von Einzelkomponenten kann beispielsweise eine Mehrzahl von Haarfärbekomponenten zum Erzeugen eines Haarfärbemittels, eine Mehrzahl von Haarpflegekomponenten zum Erzeugen eines Haarpflegemittels oder eine Mehrzahl von Haarstylingkomponenten zum Erzeugen eines Haarstylingmittels aufweisen.

In verschiedenen Ausführungsbeispielen kann die Mehrzahl von Komponenten mindestens eine Basiskomponente und eine Mehrzahl von Ergänzungskomponenten aufweisen, wobei die Ergänzungskomponenten in einer mittels des Verfahrens ermittelten Zusammensetzung (z.B. einem Mengenverhältnis) mit der Basiskomponente gemischt werden können.

Zum Bereitstellen des Pflegemittels kann in verschiedenen Ausführungsbeispielen eine Mehrzahl von (Ergänzungs-)Komponenten mit unterschiedlichen Pflegeleistungen in der Haarbehandlungsmittel-Mischvorrichtung bereitgestellt sein. Die Komponenten können bekannte Pflegestoffe wie quaternäre Stickstoffverbindungen (z.B. Hexadecyltrimethylammoniumchlorid), kationische Polymere (z.B. solche der INCI (International Nomenclature of Cosmetic Ingredients) Polyquaternium-10), Silikone und/oder Dicarbonsäure (z.B. Bernsteinsäure oder Maleinsäure) aufweisen. Bei den genannten Komponenten kann eine Pflegeleistung von den quaternären Stickstoffverbindungen über die kationischen Polymere und die Silikone hin zu der Dicarbonsäure zunehmen. In verschiedenen Ausführungsbeispielen können stattdessen oder ergänzend dazu andere bzw. weitere bekannte Pflegestoffe verwendet werden. Geeignete Pflegemittel umfassen insbesondere Conditioner, Haarkuren, Haarmasken oder 2in1-Shampoos.

Zum Bereitstellen des kosmetischen Haarpflegemittels kann bzw. können in verschiedenen Ausführungsbeispielen eine oder mehrere der Komponenten als Ergänzungskomponente(n) zur Basiskomponente hinzugefügt werden, um das ermittelte Haarpflegemittel zu bilden. Ein Gewichtsanteil einer jeweiligen Ergänzungskomponente kann dabei typischerweise in einem Bereich von unter 1% bis zu wenigen Prozent liegen.

Zum Bereitstellen des Haarfärbemittels kann in verschiedenen Ausführungsbeispielen eine Mehrzahl von (Ergänzungs-)Komponenten mit unterschiedlichen Farbtönen in der Haarbehandlungsmittel-Mischvorrichtung bereitgestellt sein. Dabei sind die "unterschiedlichen Farbtöne" so zu verstehen, dass die verschiedenen Komponenten auf derselben Standard-Haarprobe beim Färben zu unterschiedlichen Färbeergebnissen führen würden.

Die in der Haarbehandlungsmittel-Mischvorrichtung bereitgestellten Haarfärbemittel-Komponenten können in verschiedenen Ausführungsbeispielen bekannte Farbstoffvorstufen sein, welche erst im Haar Farbpigmente bilden, wie beispielsweise p-Toluylen-diaminsulfat, m-Aminophenol, Toluen-2,5-Diamine 4-Toluylendiamin, p-Aminophenol, Phenylenediamin, 4-Phenylendiamin und/oder andere.

Alternativ oder zusätzlich können die in der Haarbehandlungsmittel-Mischvorrichtung bereitgestellten Haarfärbemittel-Komponenten in verschiedenen Ausführungsbeispielen direktfärbende Komponenten (Direktfarbstoffe) aufweisen, d.h. solche, die keine Farbstoffvorstufen sind, sondern Farbpigmente bereits vor einem Kontakt mit dem Haar aufweisen.

In verschiedenen Ausführungsbeispielen kann die Datenverarbeitungsvorrichtung eingerichtet sein, beim Ermitteln der Zusammensetzung des Haarbehandlungsmittels zu berücksichtigen, welche Komponenten die Haarbehandlungsmittel-Mischvorrichtung bereithält.

In verschiedenen Ausführungsbeispielen können die in der Haarbehandlungsmittel-Mischvorrichtung bereitgehaltenen Farbstoffkomponenten so gewählt sein, dass ein möglichst breites Spektrum an möglichen Haarfarben durch Kombinieren der Komponenten miteinander erzielbar ist. Anders ausgedrückt können die bereitgehaltenen Farbstoffkomponenten so gewählt sein, dass ein mittels Färbens zugänglicher Farbraum eine möglichst große Fläche bzw. (je nach Parametrisierung) ein möglichst großes Volumen aufweist.

In verschiedenen Ausführungsbeispielen können die in der Haarbehandlungsmittel-Mischvorrichtung bereitgehaltenen Farbstoffkomponenten so gewählt sein, dass für besonders beliebte Haarfarbenbereiche, d.h. solche Haarfarbenbereiche, für welche eine Wahrscheinlichkeit höher ist, dass sie verlangt werden (z.B. einen Haarfarbenbereich um einen hennaroten Farbton oder ein Haarfarbenbereich um einen auberginefarbenen Farbton), eine derart feine Abstufung der mittels der durch die Haarbehandlungsmittel-Mischvorrichtung bereitgestellten Haarfärbemittels erzielbaren Haarfarben, dass zumindest für den mindestens einen beliebten Haarfarbenbereich eine vollständige Abdeckung dadurch erzielt wird, dass das Haarfärbemittel so bereitgestellt werden kann, dass ein Farbabstand zwischen dem erwarteten Haarfärbeergebnis und einer Wunschhaarfarbe immer unter einer Wahrnehmungsschwelle des Menschen liegt.

Zum Bereitstellen des Haarfärbemittels kann eine bzw. können mehrere der Komponenten in verschiedenen Ausführungsbeispielen als Ergänzungskomponente(n) zur Basiskomponente hinzugefügt werden, um das ermittelte Haarfärbemittel zu bilden.

Zum Bereitstellen des Haarstylingmittels kann in verschiedenen Ausführungsbeispielen eine Mehrzahl von (Ergänzungs-)Komponenten mit unterschiedlichen Stylingwirkungen in der Haarbehandlungsmittel-Mischvorrichtung bereitgestellt sein. Die Komponenten können bekannte Haarstylingmittelkomponenten aufweisen, z.B. zum Erzeugen einer Dauerwelle, mittels welcher das Haar beispielsweise dauerhaft gewellt oder geglättet werden kann. Eine Dauerwelle wird typischerweise zweistufig mittels Aufbringens zweier unterschiedlicher Mittel (eines Wellmittels und eines Fixiermittels) auf das Haar erzeugt. Dementsprechend kann die Haarbehandlungsmittel-Mischvorrichtung eingerichtet sein, beide Mittel zu erzeugen. Zum Erzeugen des Wellmittels kann die Haarbehandlungsmittel-Mischvorrichtung als Ergänzungskomponenten beispielsweise jeweils ein oder mehrere bekannte Reduktionsmittel, pH-Puffer, Emulgatoren usw. aufweisen. Zum Erzeugen des Fixiermittels kann die Haarbehandlungsmittel-Mischvorrichtung als Ergänzungskomponenten beispielsweise jeweils ein oder mehrere bekannte Oxidationsmittel, Emulgatoren usw. bereithalten.

In verschiedenen Ausführungsbeispielen kann das Stylingmittel ein Haarwachs oder-gel, ein Haarspray, einen Haarschaum, einen Straightener oder ähnliches aufweisen, welche analog zu dem, was oben für das Pflegemittel, das Färbemittel und die Dauerwellemittel beschrieben ist, aus bekannten Komponenten individuell gemischt werden können.

In verschiedenen Ausführungsbeispielen kann das Stylingmittel so zusammengestellt werden, dass trotz einer zuverlässigen Stylingwirkung eine (weitere) Haarschädigung minimiert ist.

In verschiedenen Ausführungsbeispielen kann das System um weitere Elemente erweitert sein, beispielsweise um eine weitere Analysevorrichtung für den Haarzustand, insbesondere einen Haarstatus, z.B. ein Photometer/Colorimeter oder eine Farbkarte, oder/und um eine weitere Mischmaschine für ein individualisiertes Haarbehandlungsmittel, z.B. ein Haarfärbemittel.

In verschiedenen Ausführungsbeispielen können bei dem Ermitteln der Zusammensetzung des Haarbehandlungsmittels (z.B. des Haarpflege-/ Haarfärbe-/ und/oder Haarstylingmittels) Daten und/oder Erfahrungswerte von (weiteren) Nutzern, welche einen ähnlichen Haarzustand (z.B. einen ähnlichen Schädigungsgrad und/oder eine ähnliche Haarfarbe) und ggf. ein ähnliches Profil (Alter, Geschlecht, Lebensgewohnheiten, Haartyp, Ethnizität, etc.) aufweisen können, berücksichtigt werden. Ein breiter Daten- und/oder Erfahrungssatz kann dabei zu Hilfe genommen werden, um das Ergebnis zu optimieren. In einer Cloud-basierten Ausführung des Verfahrens kann der Friseur auf Daten und/oder Erfahrungen von weiteren Nutzern zurückgreifen, die nicht notwendigerweise von ihm behandelt wurden und/oder Kunden seines Salons sind. Das System kann ggf. als ein lernendes System gestaltet sein.

In verschiedenen Ausführungsbeispielen kann das Haarbehandlungsmittel bereitgestellt werden, ohne dass der Nutzer (z.B. regelmäßiger) Kunde eines Friseurs/Salons ist.

In verschiedenen Ausführungsbeispielen kann mittels des Systems, z.B. mittels der Sensorvorrichtung in Verbindung mit der Datenverarbeitungsvorrichtung, eine standardisierte und objektive Beurteilung des Behandlungsergebnisses ermöglicht sein. Zu dem Zweck kann mittels des mindestens einen Sensors der Sensorvorrichtung der Haarzustand des Nutzers nach der Behandlung mit dem angefertigten Haarbehandlungsmittel ermittelt werden.

Erfindungsgemäß ist das System zum Bereitstellen des Haarbehandlungsmittels so eingerichtet, dass mittels der Sensorvorrichtung ein Ermitteln eines Grads der wichtigsten, nämlich der oxidativen Haarschädigung, durch eine Bestimmung eines Gehaltes an Cysteinsäure, exakt ermittelbar ist. Der Sensor ist dabei mindestens ein optischer Sensor, welcher eingerichtet sein kann, eine oder mehrere Aufnahmen in einem Fluoreszenzbereich und/oder in einem Nahinfrarotbereich (NIR-Bereich) zu machen.

Der Fluoreszenzbereich kann in verschiedenen Ausführungsbeispielen ein Wellenlängenbereich sein, in welchem geschädigtes Haar Eigenfluoreszenz emittiert und/oder ein Wellenlängenbereich, in welchem Fluoreszenzfarbstoffe, welche von geschädigtem Haar stärker adsorbiert werden als von ungeschädigtem Haar, Fluoreszenzlicht emittieren.

Der Nahinfrarotbereich kann in verschiedenen Ausführungsbeispielen ein Wellenlängenbereich sein, in welchem geschädigtes Haar Absorptionsstrukturen aufweist, z.B. in welchem Cysteinsäure Licht absorbiert. Bei der Nahinfrarotspektroskopie findet die Detektion im nahen Infrarot (780-2500 nm oder ca. 12.800-4.000 cm⁻¹) statt. Im Folgenden wird für Licht mit einer Wellenzahl in einem Bereich von 12.800 bis 4000 cm⁻¹ der Begriff Nahinfrarot (NIR) und für Licht mit einer Wellenzahl in einem Bereich von 3999 bis 400 cm⁻¹ der Begriff Infrarot (IR) verwendet.

Ungeschädigtes Haar kann typischerweise einen Cysteinsäuregehalt in einem Bereich von etwa 0.5% bis etwa 1% (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15% (Gew.) ansteigen.

In verschiedenen Ausführungsbeispielen wird diese Eigenschaft genutzt, um den Schädigungsgrad des Haars als einen Gehalt an Cysteinsäure zu quantifizieren.

In verschiedenen Ausführungsbeispielen kann geschädigtes Haar eine Eigenfluoreszenz zeigen, welche genutzt wird, um mittels Erfassens einer Fluoreszenzintensität des Haars den Schädigungsgrad zu ermitteln.

In verschiedenen Ausführungsbeispielen kann das Haar mit einer Fluoreszenzfarbstofflösung benetzt werden, welche von geschädigtem Haar besser adsorbiert wird als von ungeschädigtem Haar, wobei die Fluoreszenzfarbstofflösung Rhodamin B, Cumarin und/oder Fluoreszein aufweisen kann.

In verschiedenen Ausführungsbeispielen kann zum Ermitteln der Fluoreszenzintensität des Haars das Haar mit UV-Licht (z.B. mit Licht in einem Wellenlängenbereich von etwa 315 nm bis etwa 380 nm) belichtet werden. Zu dem Zweck kann die Sensorvorrichtung mit einer UV-Lichtquelle versehen sein. Die UV-Lichtquelle kann eine UV-LED oder eine andere geeignete Lichtquelle sein, z.B. eine herkömmliche UV-Lampe, wie sie bei einer Echtheitsprüfung von Geldscheinen verwendet wird.

Während des Belichtens kann Fluoreszenzlicht registriert werden, welches von dem Haar emittiert wird. Die Fluoreszenzintensität kann anhand des registrierten Lichts ermittelt werden. Unter Einbeziehung der Fluoreszenzintensität des Haars kann dann der Schädigungsgrad des Haars ermittelt werden.

Dementsprechend kann die Sensorvorrichtung in verschiedenen Ausführungsbeispielen einen optischen Sensor aufweisen, der zumindest im Fluoreszenz-Wellenlängenbereich empfänglich ist, beispielsweise eine Kamera, ein Photometer, ein Colorimeter und/oder ein Spektrometer. In verschiedenen Ausführungsbeispielen kann zwischen dem Haar und dem optischen Sensor in verschiedenen Ausführungsbeispielen ein Filter angeordnet sein.

In verschiedenen Ausführungsbeispielen kann das Nahinfrarot- (NIR-) und/oder ein Infrarot- (IR-)Spektrum gewonnen werden, beispielsweise mittels ATR-(Nah-)Infrarotspektroskopie (von Englisch "attenuated total reflection", auf Deutsch "abgeschwächte Totalreflexion"). Durch eine Anwendung von mathematischen Modellen kann mittels Vermessung von Kalibrier-Haarproben, welche einen anhand eines bekannten analytischen Verfahrens ermittelten Cysteinsäuregehalt aufweisen, ein mathematisches Modell erstellt werden.

Bei einer Analyse eines am Haar des Verbrauchers aufgenommenen NIR- bzw. IR-Spektrums, bzw. zumindest eines Teils davon, kann in verschiedenen Ausführungsbeispielen das Modell eine Berechnung des Gehalts an Cysteinsäure, und damit der Haarschädigung, erlauben. Eine Analyse von zumindest einem Teil des Spektrums und eine Anwendung des Modells kann dabei mittels der Datenverarbeitungsvorrichtung, beispielsweise (mit geeigneten Apps) mittels bekannter Smartphones, Tablets o.ä., ausgeführt werden.

Die Sensorvorrichtung kann eine NIR-Lichtquelle oder/und eine IR- Lichtquelle zum Belichten des Haars mit NIR- bzw. IR-Licht aufweisen.

Das Ermitteln des Schädigungsgrads von Haar kann gemäß verschiedenen Ausführungsbeispielen entweder unter Nutzung des Nahinfrarot-Bereichs ausgeführt werden, d.h. mittels Bestrahlens des Haars mit dem Nahinfrarotlicht und Spektralanalyse von zumindest einem Teil des NIR-Lichts, nachdem dieses mit dem Haar gewechselwirkt hat, oder unter Nutzung des Infrarot-Bereichs, d.h. mittels Bestrahlens des Haars mit Infrarotlicht und Spektralanalyse von zumindest einem Teil des IR-Lichts, nachdem dieses mit dem Haar gewechselwirkt hat, oder unter Verwendung von sowohl dem Nahinfrarot- als auch dem Infrarotbereich, d.h. mittels Bestrahlens des Haars mit Nahinfrarot- und Infrarotlicht und Spektralanalyse von zumindest einem Teil des NIR- und zumindest einem Teil des IR-Lichts, nachdem dieses mit dem Haar gewechselwirkt hat.

In verschiedenen Ausführungsbeispielen kann ein vermessener Nahinfrarot (NIR)-Bereich Wellenzahlen von etwa 12.800 cm⁻¹ bis etwa 4000 cm⁻¹, z.B. von etwa 5022 cm⁻¹ bis etwa 4020 cm⁻¹aufweisen. Dieser Wellenlängenbereich kann u.a. charakteristische Oberton- und Kombinationsschwingungen von z.B. CH-, OH- und NH-Gruppen aufweisen.

In verschiedenen Ausführungsbeispielen kann der zumindest eine Teil des Nahinfrarot- und/oder Infrarotlichts einen (Infrarot-)Wellenzahlbereich von etwa 1100 cm⁻¹ bis etwa 1000 cm⁻¹ z.B. um etwa 1040 cm⁻¹, aufweisen. Hier können sich u. a. die relevanten Absorptionsbanden der zu analysierenden Komponente Cysteinsäure befinden.

In verschiedenen Ausführungsbeispielen kann anhand von Ergebnissen einer quantitativen rechnergestützten Auswertung (auch als chemometrischen Analyse bezeichnet) für eine Mehrzahl von Kalibrationshaarproben in Kombination mit mittels eines unabhängigen Verfahrens, z.B. mittels Hochdruckflüssigchromatographie, für dieselben Kalibrationshaarproben gewonnenen Werten für einen Cysteinsäuregehalt der jeweiligen Kalibrationshaarprobe, ein Kalibrationsmodell erstellt werden.

Liegt das Kalibrationsmodell vor, kann in verschiedenen Ausführungsbeispielen sehr einfach für das zu messende Haar anhand des für das aufgenommene (N)IR-Spektrum die Konzentration der Cysteinsäure (als Maß für die Haarschädigung) aus den Spektren im Vergleich mit den Kalibrationsspektren berechnet werden.

Genauso kann für andere Haarinhaltsstoffe, wie beispielsweise Aminosäuren, Lipiden, Wasser oder Melamin, ein Kalibrationsmodell erstellt werden und für das zu messende Haar anhand des für das aufgenommene (N)IR-Spektrum die Konzentration der Haarinhaltsstoffs zur Bestimmung des Haarstatus aus den Spektren im Vergleich mit den Kalibrationsspektren berechnet werden.

In verschiedenen Ausführungsbeispielen kann das Ermitteln des Haarzustands, insbesondere des Schädigungsgrads, des Haars ein Ermitteln einer Oberflächenschädigung des Haars mittels Interferenzreflexionsmikroskopie aufweisen.

Mit Hilfe der Interferenzreflexionsmikroskopie können sehr dünne Haarstrukturen untersucht werden. Die Interferenzmikroskopie beruht auf der Bildung von Interferenzen, die entstehen, wenn Licht an der oberen und der unteren Grenzfläche einer Struktur reflektiert wird und reflektiertes Licht von beiden Grenzflächen miteinander interferiert. Dadurch entstehen Interferenzmuster, die beobachtet werden können, und die Aufschluss über die Dicke der Struktur liefern. Die entstehenden Interferenzfarben lassen Strukturmessungen im Bereich unter 200 nm zu. Durch Betrachten der Interferenzfarben durch ein Lichtmikroskop können diese Strukturmessungen entsprechend zu mikroskopisch erkennbaren Strukturen zugeordnet werden.

Gemäß verschiedenen Ausführungsformen kann dies auf die Cuticula von Haaren angewendet werden, um den Schädigungsgrad der Haare zu ermitteln.

Dabei kann während eines Belichtens des Haars mit Licht von dem Haar abgestrahltes Licht registriert werden. Basierend auf dem registrierten Licht können erste Bereiche des Haars ermittelt werden, die das Licht mit höherer Interferenz reflektieren und deshalb in einer Aufnahme des Lichts heller erscheinen, und zweite Bereiche des Haars, die das Licht mit niedrigerer Interferenz reflektieren und deshalb dunkler erscheinen. Basierend auf den Größen der ersten Bereiche und der zweiten Bereiche kann der Schädigungsgrad des Haars ermittelt werden.

Das Haar weist eine Cuticula, einen Kortex und Mark auf.

Wird das Haar mit Licht (z.B. Weißlicht) bestrahlt, welches mittels einer Lichtquelle der Sensorvorrichtung bereitgestellt werden kann, z.B. einer Weißlicht-LED, so wird ein Teil des Lichts an der Außenfläche der Cuticula reflektiert und ein Teil wird an der Grenzfläche zwischen Cuticula und Kortex reflektiert (insbesondere dann, wenn die Cuticula sich von dem Kortex abgehoben oder abgelöst hat, was typischerweise einer Schädigung des Haares entspricht). Die reflektierten Teile interferieren und bilden ein Interferenzmuster.

Die Sensorvorrichtung kann eine Kamera aufweisen, die an ein Interferenzmikroskop der Sensorvorrichtung gekoppelt sein kann. Das Interferenzmikroskop, welches einen Vergrößerungsfaktor in einem Bereich von etwa 10-1000 aufweisen kann, z.B. etwa 200-400, kann auf das Haar gerichtet sein und das vom Haar, z.B. von einer oder mehreren Haarfasern, reflektierte Licht auf einen Detektor der Kamera abbilden, welcher das reflektierte Licht als mindestens ein (digitales) Foto registrieren kann.

Die Datenverarbeitungsvorrichtung kann in verschiedenen Ausführungsbeispielen für das bzw. jedes der Fotos Art und/oder Anzahl der Interferenz-Muster der Haare mittels einer Bildanalyse-Software ermitteln, sie mit einem auf gleiche Art erstellten Kalibriermodell vergleichen und so einen Schädigungsgrad der Haare ermitteln. Bei mehreren Fotos kann die Datenverarbeitungsvorrichtung beispielsweise einen Mittelwert der für die Fotos ermittelten Schädigungsgrade bilden.

Das Interferenzmikroskop, die Kamera, die Datenverarbeitungsvorrichtung und gegebenenfalls auch die Lichtquelle können in verschiedenen Ausführungsbeispielen durch ein Smartphone realisiert werden, das mit einem Mikroskop-Objektiv für Smartphones ausgestattet ist, das auch für Interferenzmikroskopie geeignet ist. Um stabile Untersuchungsbedingungen zu gewährleisten, können die Haare in verschiedenen Ausführungsbeispielen auf einem Träger angeordnet sein bzw. werden. Dieser kann beispielsweise mittels der Datenverarbeitungsvorrichtung (z.B. des Smartphones) oder eines Aufsatzes für die Datenverarbeitungsvorrichtung bereitgestellt werden.

Beispielsweise können mittels eines tragbaren elektronischen Geräts (wie beispielsweise eines Smartphones, eines Tablets, etc.) mit Mikroskop-Aufsatz (wie beispielsweise einem Scrona µpeek) in Kombination mit einem Interferenz-Schieber (engl. interference slider, wie beispielsweise angeboten von Hirox Ltd.) Haare bei 350fachen Vergrößerung aufgenommen werden.

Der Flächenteil der interferierenden Haarstrukturen, das heißt der Anteil der hellen (geschädigten) Bereiche, an dem Gesamtbereich des Haars in dem Foto, liegt beispielsweise zwischen 1% und 50%, z.B. zwischen 5% und 30%.

Die Datenverarbeitungseinrichtung kann dann von dem ermittelten Flächenanteil heller Flächen auf den Schädigungsgrad schließen, beispielsweise mit Hilfe einer Tabelle, die Bereiche von Flächenanteilen Schädigungsgraden zuordnet.

Eine Art der Schädigung, welche mittels der Interferenzreflexionsmikroskopie ermittelbar ist, kann beispielsweise eine mechanische Schädigung sein, wie sie z.B. durch Dehnung des Haars verursacht werden kann.

In verschiedenen Ausführungsbeispielen können für die Quantifizierung des Cysteinsäuregehalts (z.B. mittels Fluoreszenzanalyse und/oder mittels (N)IR-Spektroskopie) bzw. des Schädigungsgrads (z.B. mittels (N)IR-Spektroskopie, mittels Interferenzreflexionsmikroskopie und/oder mittels akustischer Analyse) geeignete mathematische Modelle der Prädiktiven Analytik genutzt werden.

In verschiedenen Ausführungsbeispielen wird ein in einer Nutzung einfaches Verfahren zum Bereitstellen eines Haarbehandlungsmittels bereitgestellt, welches mit Hilfe von Fluoreszenz-Detektion und/oder durch Detektion von Absorption und/oder durch Detektion einer Oberflächenschädigung des Haars und Methoden aus der Prädiktiven Analytik eine präzise Ermittlung eines Haarzustands, insbesondere eines Grads einer (oxidativen) Schädigung von Haar oder eines Haarzustands, ermöglicht.

In verschiedenen Ausführungsbeispielen kann das Ermitteln des Haarzustands aufgrund seiner einfachen experimentellen Durchführbarkeit geeignet sein für eine Ausführung unter Verwendung einer tragbaren Datenverarbeitungsvorrichtung, auch als mobile Datenverarbeitungsvorrichtung bezeichnet. Als tragbare Datenverarbeitungsvorrichtung kann dabei beispielsweise ein Smartphone, ein iPad, ein Tablet oder Laptop genutzt werden.

In verschiedenen Ausführungsbeispielen kann ein Verfahren bereitgestellt werden, welches es ermöglicht, mittels einfacher bildanalytischer Verfahren, welche sich beispielsweise unter Verwendung einer mobilen Datenverarbeitungsvorrichtung (z.B. eines Smartphones), weniger weiterer einfacher Vorrichtungen (z.B. einer UV-LED, einer Weißlicht-, NIR- und/oder IR-Leuchtvorrichtung, eines Filters, eines tragbaren NIR-Sensors, eines tragbaren (NIR- und/oder VIS-) Spektrometers, eines Mikrofonkamms und/oder eines Mikroskops) und eines Prädiktive-Analytik-Verfahrens verwirklichen lassen, ein individualisiertes Haarbehandlungsmittel bereitzustellen.

In verschiedenen Ausführungsbeispielen kann das individualisierte Haarbehandlungsmittel geeignet sein zum Erzielen einer gewünschten Wirkung (z.B. Haarfarbe, Haarpflegezustand, Haarstatus und/oder Haarstyling).

In verschiedenen Ausführungsbeispielen kann eine Intensität einer Absorption des (N)IR-Lichts durch einen Haarinhaltsstoff, insbesondere durch Wasser oder Cysteinsäure, oder eine Intensität von Fluoreszenzlicht mit Hilfe von bildanalytischen Verfahren unter normierten Bedingungen einfach erfasst werden. Durch eine Anwendung von mathematischen Modellen aus dem Bereich Prädiktive Analytik kann mittels Vermessung von Standard-Haarproben, welche einen anhand bekannter aufwändiger Verfahren ermittelten Gehalt an Haarinhaltsstoff aufweisen, ein mathematisches Modell erstellt werden, welches dann bei den Haaren des Verbrauchers anhand der registrierten (N)IR-Absorption oder des registrierten Fluoreszenzlichts und der daraus ermittelten Fluoreszenzintensität eine Berechnung eines Gehalts an Haarinhaltsstoff, insbesondere an Cysteinsäure oder Wasser, und damit der Haarschädigung und/oder des Haarstatus, erlaubt. Die Bildanalyse kann dabei beispielsweise (mit geeigneten Apps) mittels bekannter Smartphones, Tablets o.ä. ausgeführt werden.

In verschiedenen Ausführungsbeispielen erlaubt die Verwendung der mathematischen Modelle aus dem Bereich Prädiktive Analytik (wie z.B. Tree Ensembles, neuronale Netze oder Support Vector Machines) eine wesentlich exaktere Berechnung des Haarzustands, insbesondere der Haarschädigung und/oder des Haarstatus (welche in den Modellen eine abhängige Variable bilden), als dies mit einfachen Modellen, z.B. einer einfachen linearen Regression, möglich wäre. Die Verfahren können dabei eine Vielzahl von Input-Variablen parallel nutzen und auch nichtlineare Zusammenhänge abbilden. In verschiedenen Ausführungsbeispielen ermöglichen diese Modelle beispielsweise eine Einbeziehung kategorialer, nicht-metrischer Input-Variablen, wie z.B. einer Haarfarbe (z.B. blond, braun, schwarz, usw.) und/oder ethnischer Zugehörigkeit eines Haartyps (z.B. kaukasisch, asiatisch, afro-amerikanisch), welche einen Einfluss auf eine resultierende Fluoreszenzintensität haben können. Die Input-Variablen können in verschiedenen Ausführungsbeispielen mittels der Sensorvorrichtung erfasst werden, z.B. die Haarfarbe unter Verwendung einer Kamera (z.B. der Smartphone-Kamera), eines Colorimeters oder einer Farbkarte, der Haartyp unter Verwendung der Kamera.

In verschiedenen Ausführungsbeispielen kann eine Kameraaufnahme des Haars genutzt werden, um mittels Bildanalyseverfahren nicht nur die Haarfarbe, sondern zusätzlich eine Haarstruktur zu ermitteln. Anhand einer Kombination von Haarfarbe und Haarstruktur kann gegebenenfalls die ethnische Zugehörigkeit, z.B. schwarz/kraus: afro-amerikanisch, schwarz-glatt: asiatisch, usw.) ermittelt werden.

Gemäß verschiedenen Ausführungsformen kann die Mehrzahl von NIRabsorptionsbeeinflussenden oder fluoreszenzintensitätbeeinflussenden Parametern eine Haarfarbe und/oder eine ethnische Zugehörigkeit eines Typs des Haars aufweisen.

Gemäß verschiedenen Ausführungsformen kann die prädiktive Analytik mindestens ein Verfahren nutzen aus einer Gruppe von Verfahren, wobei die Gruppe von Verfahren aufweist:
lineare oder multi-lineare Regression, polynome Regression, neuronale-Netze-Verfahren, Support Vector Machine-Verfahren, Entscheidungsbäume-Verfahren ("Decision Trees", "Random Forest", "Tree Ensembles") und weitere Verfahren.

In verschiedenen Ausführungsbeispielen kann die äußere Haarschädigung mittels einer Sensorvorrichtung, die einen Sensor zum Erfassen akustischer Emissionen, z.B. ein Mikrofon, aufweist, z.B. mittels eines Kontaktmikrofonkammes, ermittelt werden.

Hierin kann der Sensor zum Erfassen akustischer Emissionen der Einfachheit halber auch als Mikrofon bezeichnet werden. Sofern nicht anders angegeben und für die beschriebene Funktion geeignet, kann der Sensor zum Erfassen akustischer Emissionen allerdings auch beispielsweise ein Beschleunigungssensor (der geeignet sein kann, Beschleunigungen infolge akustischer Emissionen in einem gewissen Frequenzbereich zu erfassen) oder ähnliches sein.

Die Sensorvorrichtung kann in verschiedenen Ausführungsbeispielen als Kontaktmikrofonkamm ausgeführt sein. Der Kontaktmikrofonkamm kann in verschiedenen Ausführungsbeispielen einen im Wesentlichen handelsüblichen Haarkamm aufweisen, an dem ein oder mehrere von außen angebrachte Messsysteme für akustische Emissionen (d.h. Schallemissionen; als ein beispielhaftes Messsystem für akustische Emissionen kann ein Kontaktmikrofon der Fa. Korg genutzt werden) und/oder Messsonden für akustische Emissionen angeschlossen sein.

In verschiedenen Ausführungsbeispielen können mittels der Sensorvorrichtung, die das Mikrofon aufweist, Signale von erzeugtem Schall oder Vibration aufgenommen werden, die bei einem Durchkämmen von Haaren eines Nutzers entstehen.

Die Sensorvorrichtung kann in verschiedenen Ausführungsbeispielen ein oder mehrere Messsysteme für akustische Emissionen und/oder Sonden aufweisen, z.B. eines oder mehrere Kontaktmikrofone und/oder einen Beschleunigungssensor. Die Sensorvorrichtung kann in verschiedenen Ausführungsbeispielen ferner einen internen oder externen Verstärker aufweisen zum Verstärken von mittels des Messsystems für akustische Emissionen gemessenen Signalen.

In verschiedenen Ausführungsbeispielen kann eine Analyse einer Haarschädigung bereitgestellt werden mittels Digitalisierens einer Information von einem Mikrofon (und mindestens eines weiteren Sensors) und Bereitstellens dieser Daten auf einer Cloud-Plattform.

Die Information kann nach einem Verarbeiten bereitgestellt werden bzw. sein für ein Vergleichen mit bereits aufgezeichneten Beispielen (Referenzdaten). Für die Referenzdaten kann ein Haarschädigungsgrad bekannt sein, so dass als ein Ergebnis des Vergleichs beispielsweise der Haarschädigungsgrad der ähnlichsten Referenzdaten als Ergebnis in digitaler Form bereitgestellt, z.B. zurückübermittelt, werden kann.

Mittels Verwendens verschiedener Sensoren wie z.B. Linsen, Gyroskope und Beschleunigungsmesser, kann es möglich sein, eine Position der Sensorvorrichtung zu ermitteln, z.B. eine Position in einer Hand einer Person, um eine geeignete kosmetische Haarpflege (z.B. Produkte) zu ermitteln und einer unnötigen Haarschädigung, z.B. Haarverlust, vorzubeugen. Beispielsweise kann ein Beschleunigungssensor genutzt werden, um einen Beginn und/oder ein Ende eines Erfassungsvorgangs zu ermitteln. Unter der Annahme, dass ein Erfassungsvorgang typischerweise am Haaransatz beginnt und an den Haarspitzen endet, kann, ggf. in Kombination mit einer mittels des Beschleunigungssensors ermittelten Geschwindigkeit, mit welcher die tragbare Sensorvorrichtung bewegt wird, eine ortsaufgelöste Ermittlung, z.B. Haaransatz/mittlerer Bereich/Spitzen, der Haarzustandsinformation, Haarfarbe und dazugehörige Haarschädigung, ermöglicht sein. Auch kann ein Beschleunigungssensor oder ein Gyroskop verwendet werden, um die Zuverlässigkeit eines Erfassungsvorgangs mit einem optischen Sensor zu gewährleisten, in dem beispielsweise bei einer zu hohen Fortbewegungsgeschwindigkeit der tragbaren Sensorvorrichtung während des Erfassungsvorgangs, der Nutzer ein akustisches und/oder optisches Warnsignal erhält.

Im Rahmen dieser Anmeldung kann das Merkmal Beschleunigungssensor einen "klassischen" Bewegungssensor oder einen gyroskopischen Sensor umfassen.

Die Datenübermittlung der Sensorvorrichtung zur Datenverarbeitungsvorrichtung kann in verschiedenen Ausführungsbeispielen mittels Kabel oder über bekannte Datenfunkstandards (z.B. Bluetooth, WLAN, NFC, Thread, ZigBee, usw.) erfolgen.

In verschiedenen Ausführungsbeispielen wird eine Vorrichtung zum Bereitstellen eines Haarbehandlungsmittels bereitgestellt. Die Vorrichtung kann mindestens einen tragbaren Sensor zum Erfassen mindestens eines Sensorwerts an Haaren eines Nutzers aufweisen, eine Datenverarbeitungsvorrichtung zum Ermitteln eines Haarzustands des Nutzers mittels des erfassten mindestens einen Sensorwerts und zum computergestützten Ermitteln eines nutzerspezifischen Haarbehandlungsmittels unter Einbeziehung des ermittelten Haarzustands, und eine Haarbehandlungsmittel-Mischvorrichtung zum Anfertigen des ermittelten nutzerspezifischen Haarbehandlungsmittels.

In verschiedenen Ausführungsbeispielen kann das Ermitteln des Haarzustands zumindest ein Ermitteln eines Schädigungsgrads des Haars aufweisen.

In verschiedenen Ausführungsbeispielen kann das Ermitteln des Haarzustands zumindest ein Ermitteln eines Haarzustands aufweisen.

In verschiedenen Ausführungsbeispielen kann der mindestens eine Sensor mindestens einen optischen Sensor zum Ermitteln eines Haarinhaltsstoffgehalts, insbesondere eines Cysteinsäuregehalts des Haars, und/oder zum Ermitteln einer Haarfarbe des Nutzers und/oder zum Ermitteln einer mechanisch herbeigeführten Schädigung des Haars aufweisen.

In verschiedenen Ausführungsbeispielen kann der mindestens eine Sensor ein Mikrofon zum Ermitteln einer Oberflächenrauhigkeit des Haars aufweisen.

In verschiedenen Ausführungsbeispielen kann die Datenverarbeitungsvorrichtung Teil eines Smartphones, eines Tablets oder eines Smart Mirrors sein.

In verschiedenen Ausführungsbeispielen kann die Haarbehandlungsmittel-Mischvorrichtung eine Mischvorrichtung zum Erzeugen eines Haarpflegemittels, eines Haarfärbemittels und/oder eines Haarstylingmittels aufweisen. Unter einem Haarfärbemittel ist ein Mittel zum Ändern einer Haarfarbe zu verstehen. Das Haarfärbemittel kann also entweder eine Coloration zum Erzeugen einer Haarfarbe (z.B. schwarz, braun oder rot) sein, oder ein Blondierungsmittel zum Aufhellen/Entfernen einer Haarfarbe durch Zerstörung der Melanine.

In verschiedenen Ausführungsbeispielen können die Sensorvorrichtung und die Datenverarbeitungsvorrichtung eine integrierte Vorrichtung bilden.

In verschiedenen Ausführungsbeispielen können die Datenverarbeitungsvorrichtung und die Haarbehandlungsmittel-Mischvorrichtung eine integrierte Vorrichtung bilden.

In verschiedenen Ausführungsbeispielen können der mindestens eine Sensor und/oder die Datenverarbeitungsvorrichtung eine Vorrichtung zur kabellosen Datenübertragung aufweisen.

In verschiedenen Ausführungsbeispielen kann die Vorrichtung eine UV-Lampe zum Belichtens des Haars mit UV-Licht aufweisen, wobei der mindestens eine Sensor eine Vorrichtung zum Registrieren von Fluoreszenzlicht, welches von dem Haar emittiert wird, aufweisen kann. Das Fluoreszenzlicht kann eine Eigenfluoreszenz des geschädigten Haars sein, und/oder eine Fluoreszenz von in der Haarprobe adsorbiertem Fluoreszenzfarbstoff. Das Haar kann vom Kopf entfernt worden sein, beispielsweise zum Benetzen des Haars mit einer Fluoreszenzfarbstofflösung und/oder für das Registrieren des Fluoreszenzlichts, oder das Haar kann am Kopf des Nutzers verbleiben, beispielsweise bei einem Registrieren der Eigenfluoreszenz des Haars.

In verschiedenen Ausführungsbeispielen kann das Ermitteln des Haarzustands ein computergestütztes Ermitteln von mittels einer Mehrzahl von Haarbehandlungsmitteln erzielbaren Behandlungsergebnissen mittels prädiktiver Analytik unter Einbeziehung des ermittelten Haarzustands und ein Auswählen des nutzerspezifischen Haarbehandlungsmittels anhand der ermittelten Behandlungsergebnisse aufweisen.

In verschiedenen Ausführungsbeispielen wird ein Verfahren zum Bereitstellen eines Haarbehandlungsmittels mittels einer Vorrichtung zum Ermitteln des Haarbehandlungsmittels bereitgestellt. Das Verfahren kann ein Erfassen mindestens eines Sensorwerts an Haaren eines Nutzers mittels mindestens eines tragbaren Sensors aufweisen, ein Ermitteln eines Haarzustands des Nutzers mittels des erfassten mindestens einen Sensorwerts, wobei das Ermitteln des Haarzustands zumindest ein Ermitteln eines Schädigungsgrads des Haars und/oder eines Haarstatus aufweist, ein computergestütztes Ermitteln eines nutzerspezifischen Haarbehandlungsmittels unter Einbeziehung des ermittelten Haarzustands, und ein Anfertigen des ermittelten nutzerspezifischen Haarbehandlungsmittels mittels einer Haarbehandlungsmittel-Mischvorrichtung.

In verschiedenen Ausführungsbeispielen kann das Ermitteln des Schädigungsgrads des Haars ein Ermitteln eines Cysteinsäuregehalts des Haars aufweisen.

In verschiedenen Ausführungsbeispielen kann das Ermitteln des Cysteinsäuregehalts des Haars ein Ermitteln einer Fluoreszenzintensität aufweisen.

In verschiedenen Ausführungsbeispielen kann das Ermitteln des Cysteinsäuregehalts des Haars ein Ermitteln einer Absorptionsintensität aufweisen, beispielsweise in einem NIR-Spektralbereich.

In verschiedenen Ausführungsbeispielen kann das Ermitteln des Haarzustands ein Ermitteln eines Gehalts eines Haarinhaltsstoffes aufweisen.

In verschiedenen Ausführungsbeispielen kann das Ermitteln eines Gehalts eines Haarinhaltsstoffes ein Ermitteln eines Gehalts einer Aminosäure, eines Lipids, eines Melanins und/oder von Wasser aufweisen.

In verschiedenen Ausführungsbeispielen kann das Ermitteln des Gehalts eines Inhaltsstoffes des Haars ein Ermitteln einer Absorptionsintensität aufweisen, beispielsweise in einem VIS-, NIR- und/oder IR-Spektralbereich.

In verschiedenen Ausführungsbeispielen kann das Ermitteln des Schädigungsgrads des Haars ein Ermitteln einer Oberflächenrauhigkeit des Haars aufweisen.

In verschiedenen Ausführungsbeispielen kann das Ermitteln des Schädigungsgrads des Haars ein Ermitteln einer Oberflächenschädigung des Haars mittels Interferenzmikroskopie aufweisen.

In verschiedenen Ausführungsbeispielen kann das Ermitteln eines Haarstatus ein Ermitteln einer Haarfarbe des Nutzers aufweisen.

In verschiedenen Ausführungsbeispielen kann das Verfahren ferner ein Bereitstellen eines Behandlungsziels durch den Nutzer aufweisen.

In verschiedenen Ausführungsbeispielen kann das Behandlungsmittel ein Haarfärbemittel, ein Haarpflegemittel und/oder ein Haarstylingmittel aufweisen.

In verschiedenen Ausführungsbeispielen kann das Verfahren ferner ein Übermitteln des erfassten Sensorwerts von dem Sensor an eine Datenverarbeitungsvorrichtung und/oder Übermitteln des ermittelten Haarbehandlungsmittels von der Datenverarbeitungsvorrichtung an die Haarbehandlungsmittel-Mischvorrichtung aufweisen.

Die ermittelten Behandlungsergebnisse können in verschiedenen Ausführungsbeispielen mittels einer Anzeigevorrichtung angezeigt werden.

Ausführungsbeispiele der Erfindung sind in den Figuren dargestellt und werden im Folgenden näher erläutert.

Es zeigen
Figur 1, 2A bis 2K, 3A bis 3M und 4A bis 4K zeigen jeweils eine schematische Darstellung einer Vorrichtung zum Bereitstellen eines Haarbehandlungsmittels gemäß verschiedenen Ausführungsbeispielen; und
Figur 5 ein Ablaufdiagramm, welches ein Verfahren zum Bereitstellen eines Haarbehandlungsmittels gemäß verschiedenen Ausführungsbeispielen darstellt.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

In der vorliegenden Beschreibung werden die Begriffe Prädiktive Analytik, Predictive Analytics, Big Data und Data Mining synonym verwendet.

Bei einem hierin genannten Smartphone ist dies, sofern aus dem Kontext nicht etwas Anderes hervorgeht, als stellvertretend zu verstehen für alle ähnlichen von tragbarer Datenverarbeitungsvorrichtung, d.h. Smartphones, Tablets, iPads, Laptops, usw. Sinngemäßes gilt für Smartphonekameras und Ähnliches.

Unter einem Haarfärbemittel ist hierin ein Mittel zum Ändern einer Haarfarbe zu verstehen. Das Haarfärbemittel kann also entweder eine Coloration zum Erzeugen einer Haarfarbe (z.B. schwarz, braun oder rot) sein, oder ein Blondierungsmittel zum Entfernen/Aufhellen einer Haarfarbe.

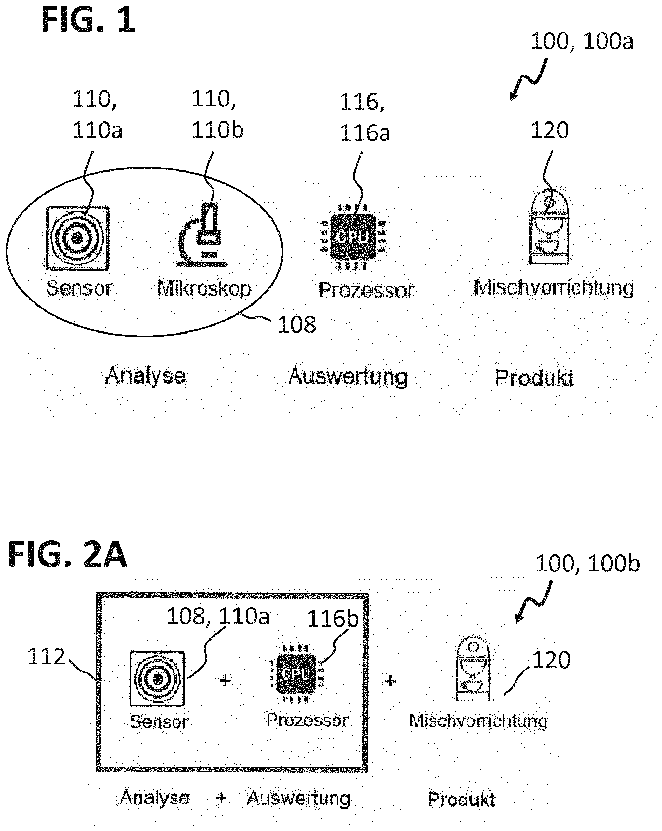

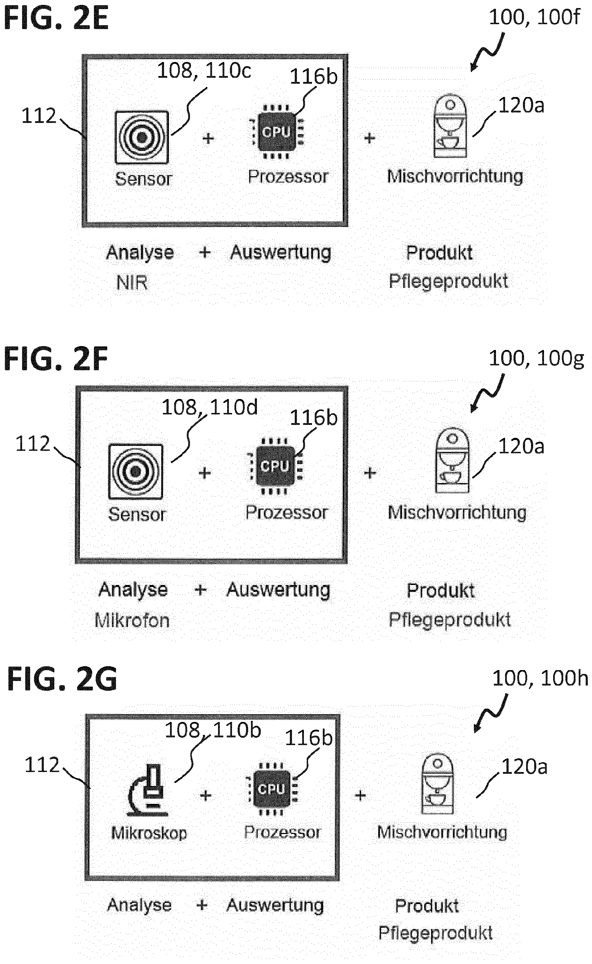

FIG. 1, 2A bis 2K, 3A bis 3M und 4A bis 4K zeigen jeweils eine schematische Darstellung einer Vorrichtung 100 zum Bereitstellen eines Haarbehandlungsmittels gemäß verschiedenen Ausführungsbeispielen. Unter die vorliegende Erfindung fallen nur die Ausführungsbeispiele, die einen optischen Sensor zur Bestimmung des Cysteinsäuregehalts aufweisen, Erindungsgemäße Ausführungsbeispiele werden daher nur in Figuren 2E, 2J, 2K, 3G, 3L, 3M, 4E, 4J und 4K gezeigt.

Unterschiedliche Ausführungsbeispiele der Vorrichtung 100 zum Bereitstellen des Haarbehandlungsmittels und anderer Vorrichtungen (Sensorvorrichtung 110, Datenverarbeitungsvorrichtung 116, Haarbehandlungsmittel-Mischvorrichtung 120) sind mit nachgestellten Buchstaben und gegebenenfalls Zahlen gekennzeichnet.

In verschiedenen Ausführungsbeispielen kann die Vorrichtung 100 eine tragbare Sensorvorrichtung 108 mit mindestens einem Sensor 110 zum Erfassen mindestens eines Sensorwerts an Haaren eines Nutzers aufweisen, eine Datenverarbeitungsvorrichtung 116 zum Ermitteln eines Haarzustands des Nutzers mittels des erfassten mindestens einen Sensorwerts, wobei das Ermitteln des Haarzustands zumindest ein Ermitteln eines Schädigungsgrads des Haars oder Ermitteln eines Haarstatus aufweist, und zum computergestützten Ermitteln eines nutzerspezifischen Haarbehandlungsmittels unter Einbeziehung des ermittelten Haarzustands, und eine Haarbehandlungsmittel-Mischvorrichtung 120 zum Anfertigen des ermittelten nutzerspezifischen Haarbehandlungsmittels.

In verschiedenen Ausführungsbeispielen kann der mindestens eine Sensor 110, wie in FIG. 1, 2B, 2D, 2E, 2G, 2J, 2K, 3B, 3D, 3F, 3G, 3J, 3L, 3M, 4B, 4D, E, 4G, 4J und 4K dargestellt, mindestens einen optischen Sensor 110b, 110c, 110e aufweisen.

Der mindestens eine optische Sensor 110b, 110c, 110e kann in verschiedenen Ausführungsbeispielen, wie in FIG. 1, 2E, 2J, 2K, 3G, 3L, 3M, 4E, 4J und 4K dargestellt, einen optischen Sensor 110c, 110e zum Ermitteln eines Haarinhaltsstoffgehalts, insbesondere eines Cysteinsäuregehalts, des Haars und/oder zum Ermitteln einer Haarfarbe des Nutzers aufweisen.

Unter einem optischen Sensor ist hierin ein Sensor zu verstehen, welcher mittels optischer Elemente Licht im weiteren Sinne (UV-Licht, sichtbares Licht (auch abkürzend als VIS bezeichnet), Nahinfrarotlicht (NIR) und/oder Infrarotlicht (IR)) leitet und mittels eines Detektors (z.B. eines elektronischen Detektors, z.B. für sichtbares Licht, für NIR- oder/und IR-Licht, eines Photometers oder Ähnlichem) erfasst.

Der optische Sensor 110e zum Ermitteln eines Haarinhaltsstoffgehalts, insbesondere eines Cysteinsäuregehalts, des Haars kann, wie oben beschrieben, in verschiedenen Ausführungsbeispielen eine Kamera und/oder ein Spektrometer zum Erfassen von Licht in einem Wellenlängenbereich sichtbaren Lichts aufweisen, wobei das Licht von dem Haar bei einem Bestrahlen mit UV-Licht als Fluoreszenzlicht abgestrahlt wird.

Bei dem optische Sensor 110e kann in verschiedenen Ausführungsbeispielen ein einzelner optischer Sensor derart eingerichtet sein, dass nur das Fluoreszenzlicht auswertbar erfasst wird, beispielsweise in einem Fall, dass zwischen dem Haar und dem optischen Sensor mindestens ein Filter angeordnet ist, welcher bzw. von welchen mindestens einer nur oder hauptsächlich einen Wellenlängenbereich des Fluoreszenzlichts passieren lässt.

Sofern vorgesehen ist, in einem solchen Fall zusätzlich die Haarfarbe mittels der Sensorvorrichtung 108 zu erfassen, kann der Filter entfernbar eingerichtet sein, beispielsweise als ein Filteraufsatz für eine Smartphonekamera, so dass das Erfassen des Fluoreszenzlichts und ein Erfassen sichtbaren Lichts in mehreren Wellenlängenbereichen (z.B. R, G, B) zum Ermitteln der Haarfarbe sequenziell erfolgen kann. Alternativ kann der Filter mehrere Bereiche aufweisen, beispielsweise einen Bandpassbereich für das Fluoreszenzlicht, ggf. einen Bandpassbereich außerhalb des Fluoreszenzlicht-Wellenlängenbereichs und/oder einen im Wesentlichen ungefilterten Bereich. Alternativ kann der mindestens eine optische Sensor 110e eine Mehrzahl optischer Sensoren 110e aufweisen, wobei mindestens einer der Sensoren zum Erfassen des Fluoreszenzlichts genutzt wird, und ein weiterer der Sensoren zum Ermitteln der Haarfarbe eingerichtet ist, beispielsweise als Kamera, z.B. eine Kamera eines Smartphones, Tablets, usw., oder z.B. als Spektrometer.

Bei dem optische Sensor 110e kann in verschiedenen Ausführungsbeispielen ein einzelner optischer Sensor derart eingerichtet sein, dass sowohl das Fluoreszenzlicht auswertbar erfasst wird, als auch ein Ermitteln der Haarfarbe des Nutzers ermöglicht ist, beispielsweise bei einer Gestaltung des optischen Sensors 110e als Spektrometer.

Der optische Sensor 110c zum Ermitteln eines Haarinhaltsstoffgehalts, insbesondere eines Cysteinsäuregehalts, des Haars kann, wie oben beschrieben, in verschiedenen Ausführungsbeispielen eine (N)IR-Kamera und/oder ein (N)IR-Spektrometer zum Erfassen von Licht in einem Wellenlängenbereich aufweisen, in welchem der Haarinhaltsstoff, insbesondere die Cysteinsäure, Licht absorbiert.

Sofern vorgesehen ist, in einem solchen Fall zusätzlich die Haarfarbe mittels der Sensorvorrichtung 108 zu erfassen, kann der mindestens eine optische Sensor 110c, 110e eine Mehrzahl optischer Sensoren 110c, 110e aufweisen, wobei mindestens einer der Sensoren 110c zum Erfassen des (N)IR-Lichts genutzt wird, und ein weiterer der Sensoren 110e zum Erfassen des sichtbaren Lichts zum Ermitteln der Haarfarbe eingerichtet ist. Alternativ kann der optische Sensor 110c ein Sensor zum Erfassen von sichtbaren Licht und (N)IR-Licht sein.

In verschiedenen Ausführungsbeispielen kann der optische Sensor 110e nur zum Erfassen eines Haarstatus, insbesondere der Haarfarbe, eingerichtet sein, und die Haarschädigung kann mittels eines anderen, z.B. akustischen, Sensors ermittelt werden.

In verschiedenen Ausführungsbeispielen kann der mindestens eine Sensor 110, wie in FIG. 1, 2B, 2D, 2E, 2G, 2J, 2K, 3B, 3D, 3F, 3G, 3J, 3L, 3M, 4B, 4D, E, 4G, 4J und 4K dargestellt, mindestens einen optischen Sensor 110b zum Ermitteln einer mechanisch herbeigeführten Schädigung des Haars (und ggf. zum Ermitteln eines Haarstatus, insbesondere einer Haarfarbe, des Nutzers) aufweisen.

Wie oben beschrieben kann die mechanisch herbeigeführte Haarschädigung mittels Interferenzreflexionsmikroskopie ermittelbar sein, wofür das Mikroskop 110b in verschiedenen Ausführungsbeispielen bereitgestellt sein kann.

In verschiedenen Ausführungsbeispielen kann, wie oben beschrieben, das Mikroskop 110b als Mikroskopaufsatz für eine Kamera, z.B. für eine Smartphonekamera, gestaltet sein. Dementsprechend kann der Aufsatz entfernbar sein, und die Smartphonekamera kann dann zum Ermitteln der Haarfarbe und/oder zum Erfassen von Fluoreszenzlicht oder (N)IR-Licht nutzbar sein.

In verschiedenen Ausführungsbeispielen kann das Mikroskop 110b als eigene Sensorvorrichtung 108 oder Teil einer Sensorvorrichtung 108, welche beispielsweise weitere Sensoren 110 aufweisen kann, gestaltet sein.

In verschiedenen Ausführungsbeispielen kann der mindestens eine Sensor 110, wie in FIG. 2F, 3H und 4F dargestellt, mindestens ein Mikrofon 110d zum Ermitteln einer Oberflächenrauhigkeit des Haars aufweisen. Die Oberflächenrauhigkeit des Haars kann in verschiedenen Ausführungsbeispielen wie oben beschrieben mittels des Mikrofons 110d ermittelt werden.

Anhand der ermittelten Oberflächenrauhigkeit kann in verschiedenen Ausführungsbeispielen der Schädigungsgrad des Haars ermittelt werden, beispielsweise wie oben beschrieben.

In verschiedenen Ausführungsbeispielen kann bzw. können das registrierte Licht und/oder das registrierte Geräusch oder ggf. ein anderer bzw. weiterer erfasster Sensorwert als Rohdaten und/oder in verarbeiteter Form, beispielsweise als Digitalfoto oder eine andere Quantifizierung des registrierten Lichts, als Audiodatei, Fouriertransformation o.ä. an die Datenverarbeitungsvorrichtung 116 übertragen werden. Dies ist, um eine Übersichtlichkeit der Figuren nicht zu beeinträchtigen, nur in FIG. 1C3 beispielhaft mit dem Bezugszeichen 114 gekennzeichnet. Auch bei den anderen Ausführungsbeispielen können jedoch die erfassten Sensorwerte in roher und/oder verarbeiteter Form der Datenverarbeitungsvorrichtung 116 zugeführt werden.

Die Übertragung kann auf bekanntem Weg erfolgen, beispielsweise mittels eines Datenkabels, kabelloser Datenübertragung (z.B. Bluetooth, WLAN, ZigBee, Thread oder Near Field Communication (NFC)), oder eine Übertragung kann innerhalb einer Vorrichtung erfolgen, wenn die Sensorvorrichtung 108, wie oben beschrieben, mit der Datenverarbeitungsvorrichtung 116 die integrierte Vorrichtung 112 bildet (z.B. bei Verwendung der Kamera und/oder des Mikrofons eines Mobiltelefons, Tablets, Laptops o.ä. als Kamera).

In verschiedenen Ausführungsbeispielen kann die Sensorvorrichtung 108 als Kamm oder Bürste gestaltet sein. Anders ausgedrückt kann der mindestens eine Sensor 110 in einen kamm- oder bürstenförmigen Körper integriert sein.

In verschiedenen Ausführungsbeispielen kann insbesondere das Mikrofon 110d in den kamm- oder bürstenförmigen Körper integriert sein, beispielsweise wie oben beschrieben.

In verschiedenen Ausführungsbeispielen können ein oder mehrere Sensoren 110, z.B. einer der optischen Sensoren 110b, 110c und/oder 110e alternativ oder zusätzlich in den kamm- oder bürstenförmigen Körper integriert sein.

In verschiedenen Ausführungsbeispielen kann die Sensorvorrichtung 108 einen, sofern zweckmäßig, im Wesentlichen beliebig geformten Körper aufweisen.

In verschiedenen Ausführungsbeispielen kann die Datenverarbeitungsvorrichtung 116 Teil eines Smartphones, Tablets, iPads etc. oder, wie in FIG. 1C1 bis 1C12 dargestellt, Teil eines Smart Mirrors 116c sein.

In verschiedenen Ausführungsbeispielen kann die Haarbehandlungsmittel-Mischvorrichtung 120 eine Mischvorrichtung 120a zum Erzeugen eines Haarpflegemittels, eine Mischvorrichtung 120b zum Erzeugen eines Haarfärbemittels und/oder eine Mischvorrichtung 120 zum Erzeugen eines Haarstylingmittels aufweisen.

In verschiedenen Ausführungsbeispielen, wie z.B. in FIG. 2A bis FIG. 2K dargestellt, können die Sensorvorrichtung 108 und die Datenverarbeitungsvorrichtung 116 eine integrierte Vorrichtung bilden.

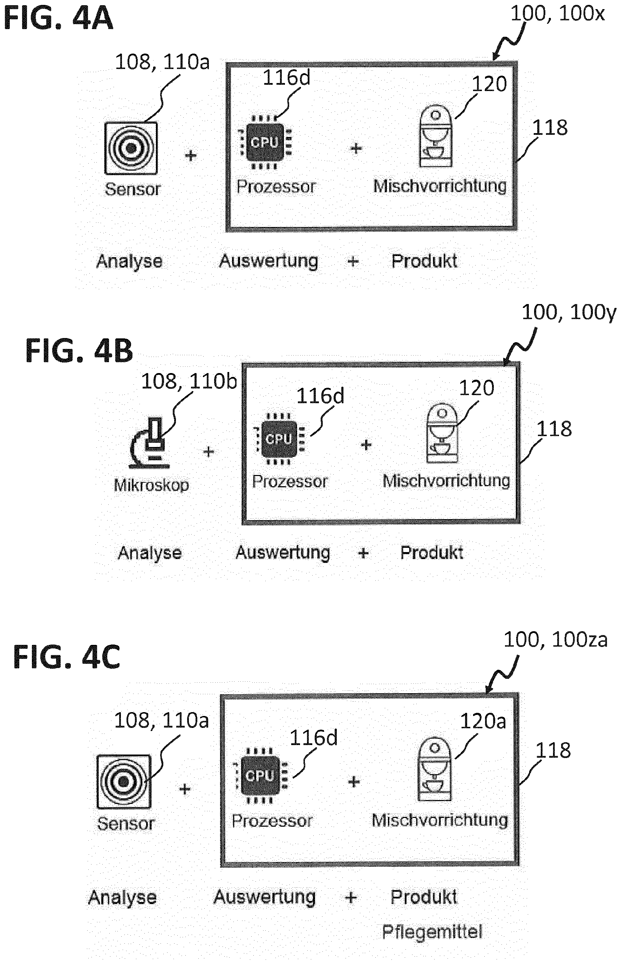

In verschiedenen Ausführungsbeispielen, wie z.B. in FIG. 4A bis 4K dargestellt, können die Datenverarbeitungsvorrichtung 116 und die Haarbehandlungsmittel-Mischvorrichtung 116 eine integrierte Vorrichtung 118 bilden.

In verschiedenen Ausführungsbeispielen können die Sensorvorrichtung 108 und/oder die Datenverarbeitungsvorrichtung 116 eine Vorrichtung zur kabellosen Datenübertragung aufweisen, beispielsweise für eine Datenübertragung mittels WLAN, Bluetooth, ZigBee, Thread, NFC oder ähnliches, z.B. wie oben beschrieben.

In verschiedenen Ausführungsbeispielen kann die Vorrichtung eine UV-Lampe zum Belichtens des Haars mit UV-Licht aufweisen, wobei die Sensorvorrichtung 108 eine Vorrichtung zum Registrieren von Fluoreszenzlicht, welches von dem Haar emittiert wird, aufweisen kann, beispielsweise wie oben beschrieben.

Das Fluoreszenzlicht kann eine Eigenfluoreszenz des geschädigten Haars sein, und/oder eine Fluoreszenz von in der Haarprobe adsorbiertem Fluoreszenzfarbstoff. Das Haar kann vom Kopf entfernt worden sein, beispielsweise zum Benetzen des Haars mit einer Fluoreszenzfarbstofflösung und/oder für das Registrieren des Fluoreszenzlichts, oder das Haar kann am Kopf des Nutzers verbleiben, beispielsweise bei einem Registrieren der Eigenfluoreszenz des Haars.

In verschiedenen Ausführungsbeispielen kann das Ermitteln des Haarzustands ein computergestütztes Ermitteln des Haarschädigungsgrads und/oder der Haarfarbe aufweisen, beispielsweise mittels prädiktiver Analytik.

In verschiedenen Ausführungsbeispielen kann das Ermitteln des nutzerspezifischen Haarbehandlungsmittels ein computergestütztes Ermitteln des Haarbehandlungsmittels, beispielsweise mittels prädiktiver Analytik, unter Einbeziehung des Haarschädigungsgrads und/oder des Haarzustands aufweisen.

Zum Empfangen und Weiterverarbeiten der Daten kann die Datenverarbeitungsvorrichtung 116 beispielsweise mit einer entsprechenden Software ausgestattet sein, beispielsweise einer App, beispielsweise wie oben beschrieben.

In verschiedenen Ausführungsbeispielen kann mittels der Datenverarbeitungsvorrichtung 116 anhand eines Maßes (z.B. einer Äquivalentbreite) für die (N)IR-Absorption oder der ermittelten Fluoreszenzintensität ein Cysteinsäuregehalt ermittelt werden. Hierfür können, z.B. wie oben beschrieben, mathematische Modelle aus dem Bereich Prädiktive Analytik genutzt werden, um eine Beziehung zwischen dem Maß für die (N)IR-Absorption oder der (standardisierten) Fluoreszenzintensität und einem zugehörigen Cysteinsäuregehalt (und damit einem Schädigungsgrad des Haars) zu ermitteln.

Als unabhängige Parameter können in das Modell dabei in verschiedenen Ausführungsbeispielen beispielsweise eine Beziehung zwischen Cysteinsäuregehalt und(N)IR-Absorption oder Cysteinsäuregehalt und Fluoreszenzintensität bzw. entsprechende Datenwerte, z.B. in Form zugeordneter Datenpaare, einbezogen werden, welche mittels Vermessung von Standard-Haarproben, welche einen anhand bekannter aufwändiger Verfahren ermittelten Cysteinsäuregehalt aufweisen, ermittelt bzw. mathematisch modelliert wurden.

Entsprechend kann in verschiedenen Ausführungsbeispielen mit anderen Sensorwerten verfahren werden, um den Schädigungsgrad des Haars und/oder den Haarstatus zu ermitteln.

In verschiedenen Ausführungsbeispielen kann der Schädigungsgrad des Haars in einer kategorialen Skala (z.B. leicht, mittel, schwer) ermittelt werden.

In verschiedenen Ausführungsbeispielen kann der Schädigungsgrad in einer metrischen Skala (z.B. Prozentanteil des Gehalts an Cysteinsäure, Prozentanteil an Flächen mit höherer Interferenz o.ä.) ermittelt werden.

Die Datenverarbeitungsvorrichtung 116 kann, wie oben beschrieben, beispielsweise eine mobile Datenverarbeitungsvorrichtung, beispielsweise ein Smartphone, ein Tablet oder einen Laptop, aufweisen, insbesondere in einem Fall, dass die tragbare Sensorvorrichtung 108 mit der Datenverarbeitungsvorrichtung 116 die integrierte Vorrichtung 112 bildet.

In verschiedenen Ausführungsbeispielen kann die Datenverarbeitungsvorrichtung von anderer Art sein, z.B. ein Desktop-Computer, integriert in den Smart Mirror, oder jede andere Datenverarbeitungsvorrichtung 116, die geeignet ist, die Daten zu speichern und bereitzustellen und das Prädiktive Analytik Verfahren auszuführen, also beispielsweise jede Datenverarbeitungsvorrichtung 116 mit hinreichend großem Datenspeicher und hinreichend leistungsfähigem Prozessor.

In verschiedenen Ausführungsbeispielen können anstelle des Prädiktive-Analytik-Verfahrens andere, z.B. einfachere, Verfahren zum Ermitteln des Haarzustands, z.B. des Schädigungsgrads oder der Haarfarbe, und/oder zum Ermitteln des nutzerspezifischen Haarbehandlungsmittels genutzt werden.

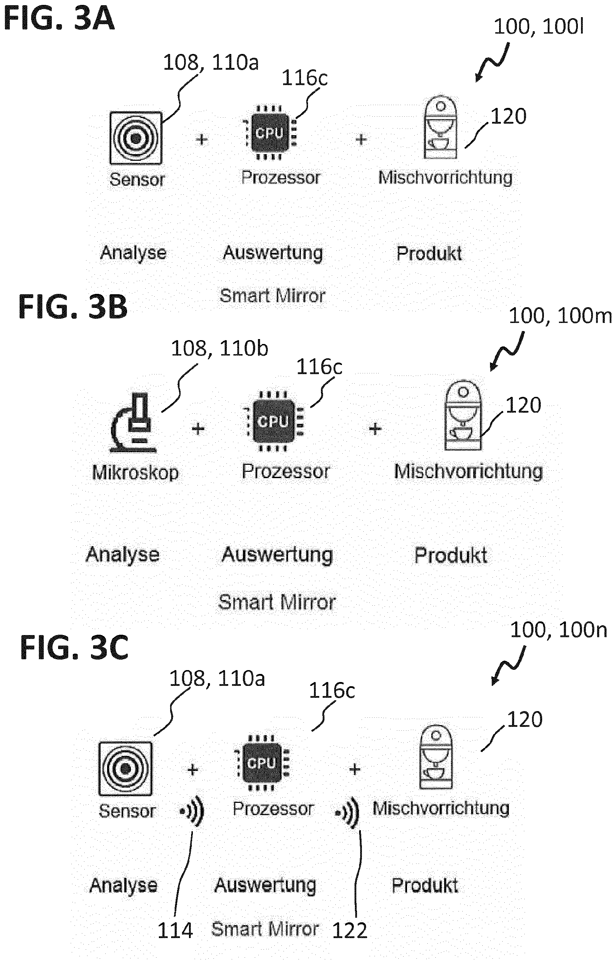

In verschiedenen Ausführungsbeispielen kann die mittels der der Datenverarbeitungsvorrichtung 116 ermittelte Rezeptur für das nutzerspezifische Haarbehandlungsmittel an die Haarbehandlungsmittel-Mischvorrichtung 120 übertragen werden. Dies ist, um eine Übersichtlichkeit der Figuren nicht zu beeinträchtigen, nur in FIG. 3C beispielhaft mit dem Bezugszeichen 122 gekennzeichnet.

Die Übertragung kann auf bekanntem Weg erfolgen, beispielsweise mittels eines Datenkabels, kabelloser Datenübertragung (z.B. Bluetooth, WLAN, ZigBee, Thread oder Near Field Communication (NFC)), oder eine Übertragung kann innerhalb einer Vorrichtung erfolgen, wenn die Datenverarbeitungsvorrichtung 116 mit der Haarbehandlungsmittel-Mischvorrichtung 120 eine integrierte Vorrichtung 118 bildet, z.B. wie oben beschrieben und in FIG. 4A bis FIG. 4K dargestellt.

In verschiedenen Ausführungsbeispielen, wie in FIG. 4A bis FIG. 4K dargestellt, können die Datenverarbeitungsvorrichtung 116 und die Haarbehandlungsmittel-Mischvorrichtung 120 die integrierte Vorrichtung 118 bilden, beispielsweise indem ein Prozessor der Haarbehandlungsmittel-Mischvorrichtung 120 eingerichtet ist, die Funktionen der Datenverarbeitungsvorrichtung 116 auszuführen, z.B. die Zusammensetzung des Haarbehandlungsmittels unter Berücksichtigung des mittels des mindestens einen Sensors 110 ermittelten Haarzustands zu ermitteln. Beispielsweise kann ein ohnehin in der Haarbehandlungsmittel-Mischvorrichtung 120 vorhandener Prozessor eingerichtet sein, die zusätzliche Funktionalität bereitzustellen, oder ein zusätzlicher Prozessor kann in die Haarbehandlungsmittel-Mischvorrichtung 120 integriert werden, um die Funktionalität bereitzustellen.

In verschiedenen Ausführungsbeispielen kann die Datenverarbeitungsvorrichtung 116 eingerichtet sein, den Haarzustand, z.B. den Haarschädigungsgrad und/oder den Haarstatus, und/oder die Zusammensetzung des Haarbehandlungsmittels indirekt zu ermitteln, beispielsweise mittels eines Übermittelns der Sensor-Rohdaten und/oder von teilweise ausgewerteten Sensordaten und/oder von einem Haarzustand an eine externe Datenverarbeitungsvorrichtung, beispielsweise an eine Cloud-Serverarchitektur (kurz: Cloud), und mittels Empfangens eines Ergebnisses von der externen Datenverarbeitungsvorrichtung (z.B. der Cloud).

In verschiedenen Ausführungsbeispielen kann die Vorrichtung zum Bereitstellen des Haarbehandlungsmittels als ein lernendes System gestaltet sein, beispielsweise indem der Nutzer und/oder weitere Nutzer den Haarzustand vor einer Anwendung des Haarbehandlungsmittels und den Haarzustand nach einem Behandeln des Haars mit dem Haarbehandlungsmittel der Datenverarbeitungsvorrichtung 116 (beispielsweise mittels der Cloud) bereitstellt/bereitstellen.

Zum Bereitstellen des Haarzustands nach der Behandlung kann in verschiedenen Ausführungsbeispielen ein Ermitteln des Haarzustands nach dem Behandeln mit dem Haarbehandlungsmittel mittels der Sensorvorrichtung 108 in Verbindung mit der Datenverarbeitungsvorrichtung 116 genutzt werden. In anderen Ausführungsbeispielen kann eine andere Vorrichtung genutzt werden, um den Haarzustand nach der Behandlung zu ermitteln, und der ermittelte Haarzustand kann der Datenverarbeitungsvorrichtung 116 übermittelt werden.

FIG. 1 stellt eine Vorrichtung 100a zum Bereitstellen eines Haarbehandlungsmittels gemäß verschiedenen Ausführungsbeispielen dar. Die Vorrichtung 100a kann als Sensoren 110 der Sensorvorrichtung 108 ein Mikroskop 110 und einen weiteren Sensor 110a aufweisen, wobei der weitere Sensor 110a einen oder mehrere der anderen beschriebenen Sensoren (optischen Sensor 110c, 110e, akustischen Sensor 110d) oder einen oder mehrere nicht beschriebene, für ein Ermitteln eines Haarzustands geeigneten Sensoren aufweisen kann. Der Sensor 110a wird im Folgenden auch als generischer Sensor 110a bezeichnet. Die Vorrichtung 100a kann eine Datenverarbeitungsvorrichtung 116a aufweisen, welche eine oder mehrere der beschriebenen Datenverarbeitungsvorrichtungen 116 aufweisen kann, z.B. ein Smartphone, einen Smart Mirror, einen Desktop-Computer oder ähnliches, oder allgemein einen Prozessor mit einem optischen oder akustischen Ein- und/oder Ausgabegerät, z.B. einem Bildschirm, einem berührungsempfindlichen Bildschirm, einem Lautsprecher und/oder einem Mikrofon. Die Vorrichtung 100a kann eine Mischvorrichtung 120 aufweisen, wobei die Mischvorrichtung 120 eingerichtet kann zum Mischen eines Haarpflegeprodukts, eines Haarfärbeprodukts und/oder eines Haarstylingprodukts (dies wird im Folgenden auch als generische Mischvorrichtung 120 bezeichnet).

FIG. 2A bis 2K stellen jeweils eine Vorrichtung 100b bis 100k zum Bereitstellen eines Haarbehandlungsmittels gemäß verschiedenen Ausführungsbeispielen dar. Dabei können jeweils die Sensorvorrichtung 108 und die Datenverarbeitungsvorrichtung 116b als eine integrierte Vorrichtung 112 gebildet sein.

Die Vorrichtung 100b aus FIG. 2A weist als integrierte Vorrichtung 112 einen generischen Sensor 110a und eine damit integrierte Datenverarbeitungsvorrichtung 116b auf, und ferner eine generische Mischvorrichtung 120. Die integrierte Vorrichtung 112 kann beispielsweise eine Bürste mit mindestens einem Sensor 110a, einen Prozessor und ein optisches oder akustisches Ein- und/oder Ausgabegerät aufweisen. Die Mischvorrichtung 120 kann separat sein.

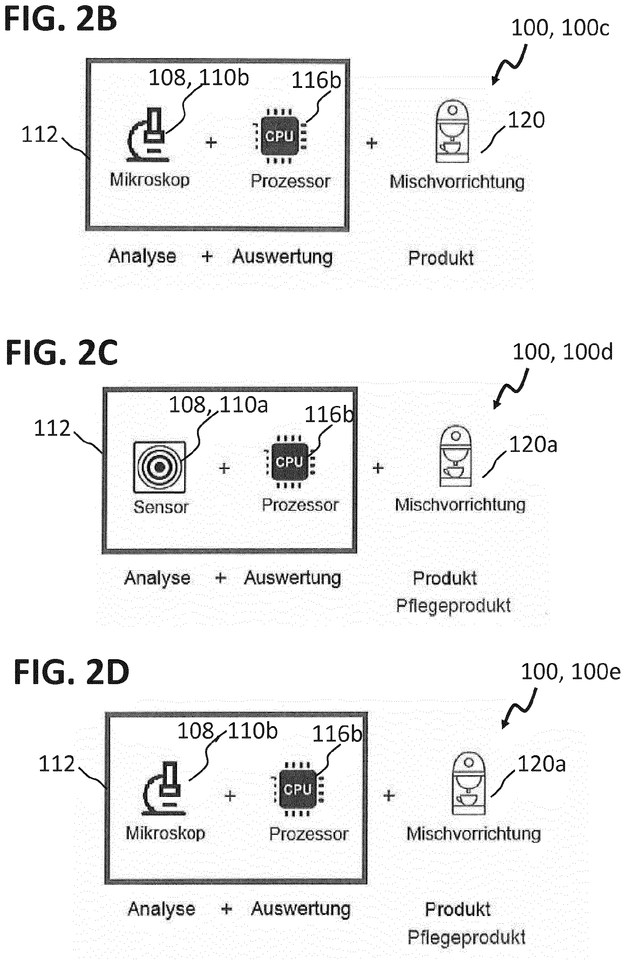

Die Vorrichtung 100c aus FIG. 2B weist als integrierte Vorrichtung 112 einen generischen Sensor 110a und eine damit integrierte Datenverarbeitungsvorrichtung 116b auf, und ferner eine generische Mischvorrichtung 120. Die integrierte Vorrichtung 112 kann beispielsweise ein smartes Endgerät mit Mikroskop(aufsatz), einen Prozessor und ein optisches oder akustisches Ein- und/oder Ausgabegerät aufweisen. Die Mischvorrichtung 120 kann separat sein.

Die Vorrichtung 100d aus FIG. 2C weist als integrierte Vorrichtung 112 einen generischen Sensor 110a und eine damit integrierte Datenverarbeitungsvorrichtung 116b auf, und ferner eine Mischvorrichtung 120a zum Erzeugen eines Pflegemittels. Die integrierte Vorrichtung 112 kann beispielsweise eine Bürste mit dem Sensor 110a, einen Prozessor und ein optisches oder akustisches Ein- und/oder Ausgabegerät aufweisen. Die Mischvorrichtung 120a kann separat sein.

Die Vorrichtung 100e aus FIG. 2D weist als integrierte Vorrichtung 112 einen generischen Sensor 110a und eine damit integrierte Datenverarbeitungsvorrichtung 116b auf, und ferner eine Mischvorrichtung 120a zum Erzeugen eines Pflegemittels. Die integrierte Vorrichtung 112 kann beispielsweise ein smartes Endgerät mit Mikroskop(aufsatz), einen Prozessor und ein optisches oder akustisches Ein- und/oder Ausgabegerät aufweisen. Die Mischvorrichtung 120a kann separat sein.

Die Vorrichtung 100f aus FIG. 2E weist als integrierte Vorrichtung 112 einen NIR-Sensor 110c zum Ermitteln der Haarschädigung und/oder zum Ermitteln des Haarstatus und eine damit integrierte Datenverarbeitungsvorrichtung 116b auf, und ferner eine Mischvorrichtung 120a zum Erzeugen eines Pflegemittels. Die integrierte Vorrichtung 112 kann beispielsweise eine Bürste mit dem Sensor 110c, einen Prozessor und ein optisches oder akustisches Ein- und/oder Ausgabegerät aufweisen. Die Mischvorrichtung 120a kann separat sein. Alternativ, nicht dargestellt, kann anstelle des NIR-Sensors 110c, ein Sensor zum Erfassen von Fluoreszenzlicht eingesetzt werden.

Die Vorrichtung 100g aus FIG. 2F weist als integrierte Vorrichtung 112 ein Mikrofon 110d zum Ermitteln der Oberflächenrauhigkeit und eine damit integrierte Datenverarbeitungsvorrichtung 116b auf, und ferner eine Mischvorrichtung 120a zum Erzeugen eines Pflegemittels. Die integrierte Vorrichtung 112 kann beispielsweise eine Bürste mit dem Mikrofon 110d, einen Prozessor und ein optisches oder akustisches Ein- und/oder Ausgabegerät aufweisen. Die Mischvorrichtung 120a kann separat sein.

Die Vorrichtung 100h aus FIG. 2G weist als integrierte Vorrichtung 112 ein(en) Mikroskop(aufsatz) 110b zum Ermitteln der Haarschädigung (z.B. Dehnungsschäden, z.B. mittels Bildauswertung) und eine damit integrierte Datenverarbeitungsvorrichtung 116b auf, und ferner eine Mischvorrichtung 120a zum Erzeugen eines Pflegemittels. Die integrierte Vorrichtung 112 kann einen Prozessor und ein optisches oder akustisches Ein- und/oder Ausgabegerät aufweisen. Die Mischvorrichtung 120a kann separat sein.

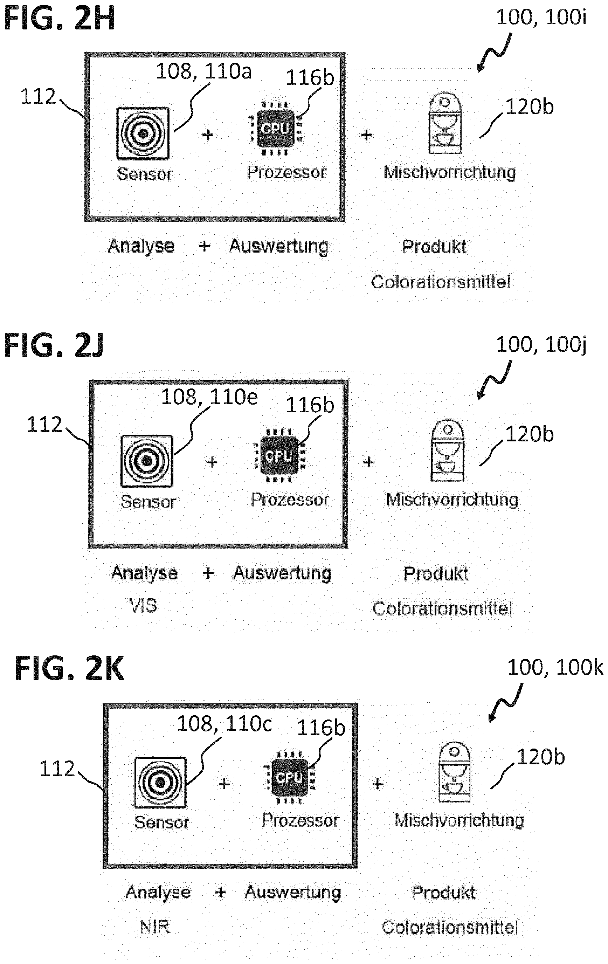

Die Vorrichtung 100i aus FIG. 2H weist als integrierte Vorrichtung 112 einen generischen Sensor 110a und eine damit integrierte Datenverarbeitungsvorrichtung 116b auf, und ferner eine Mischvorrichtung 120b zum Erzeugen eines Colorationsmittels. Die integrierte Vorrichtung 112 kann beispielsweise eine Bürste mit dem Sensor, einen Prozessor und ein optisches oder akustisches Ein- und/oder Ausgabegerät aufweisen. Die Mischvorrichtung 120b kann separat sein.

Die Vorrichtung 100j aus FIG. 2J weist als integrierte Vorrichtung 112 einen Sensor 110e für sichtbares Licht zum Ermitteln der Haarfarbe, z.B. eine digitale Farbkamera oder einen VIS-Sensor, und eine damit integrierte Datenverarbeitungsvorrichtung 116b auf, und ferner eine Mischvorrichtung 120b zum Erzeugen eines Colorationsmittels. Die integrierte Vorrichtung 112 kann einen Prozessor und ein optisches oder akustisches Ein- und/oder Ausgabegerät aufweisen. Die Mischvorrichtung 120b kann separat sein.

Die Vorrichtung 100k aus FIG. 2K weist als integrierte Vorrichtung 112 einen Sensor 110c für Nahinfrarotlicht zum Ermitteln der Haarschädigung, z.B. anhand eines Cysteinsäuregehalts, und/oder zum Ermitteln eines Haarstatus auf, und eine damit integrierte Datenverarbeitungsvorrichtung 116b auf, und ferner eine Mischvorrichtung 120b zum Erzeugen eines Colorationsmittels. Die integrierte Vorrichtung 112 kann einen Prozessor und ein optisches oder akustisches Ein- und/oder Ausgabegerät aufweisen. Die Mischvorrichtung 120b kann separat sein. Alternativ, nicht dargestellt, kann anstelle des NIR-Sensors 110c, ein Sensor zum Erfassen von Fluoreszenzlicht eingesetzt werden.

FIG. 3A bis 2M stellen jeweils eine Vorrichtung 100I bis 100w zum Bereitstellen eines Haarbehandlungsmittels gemäß verschiedenen Ausführungsbeispielen dar. Dabei können jeweils die Sensorvorrichtung 108, die Datenverarbeitungsvorrichtung 116c und die Mischvorrichtung 120 als getrennte Vorrichtungen gebildet sein. Die Datenverarbeitungsvorrichtung 116c kann Teil eines Smart Mirror sein.

Die Vorrichtung 100l aus FIG. 3A weist einen generischen Sensor 110a, eine Datenverarbeitungsvorrichtung 116c als Teil eines Smart Mirror 110c und eine generische Mischvorrichtung 120 auf. Der generische Sensor 110a kann beispielsweise eine Bürste mit mindestens einem Sensor 110a sein.

Die Vorrichtung 100m aus FIG. 3B weist ein smartes Endgerät mit Mikroskop(aufsatz) 110b, eine Datenverarbeitungsvorrichtung 116c als Teil eines Smart Mirror 110c und eine generische Mischvorrichtung 120 auf.

Die Vorrichtung 100n aus FIG. 3C weist einen generischen Sensor 110a, eine Datenverarbeitungsvorrichtung 116c als Teil eines Smart Mirror 110c und eine generische Mischvorrichtung 120 auf. Der generische Sensor 110a kann beispielsweise eine Bürste mit mindestens einem Sensor 110a sein. Daten können von dem Sensor 110a an den Smart Mirror 116c als Signal 114 übermittelt werden, und der Smart Mirror 116c kann der Mischvorrichtung 120 die Zusammensetzung des Haarbehandlungsmittels als ein Signal 118 übermitteln.

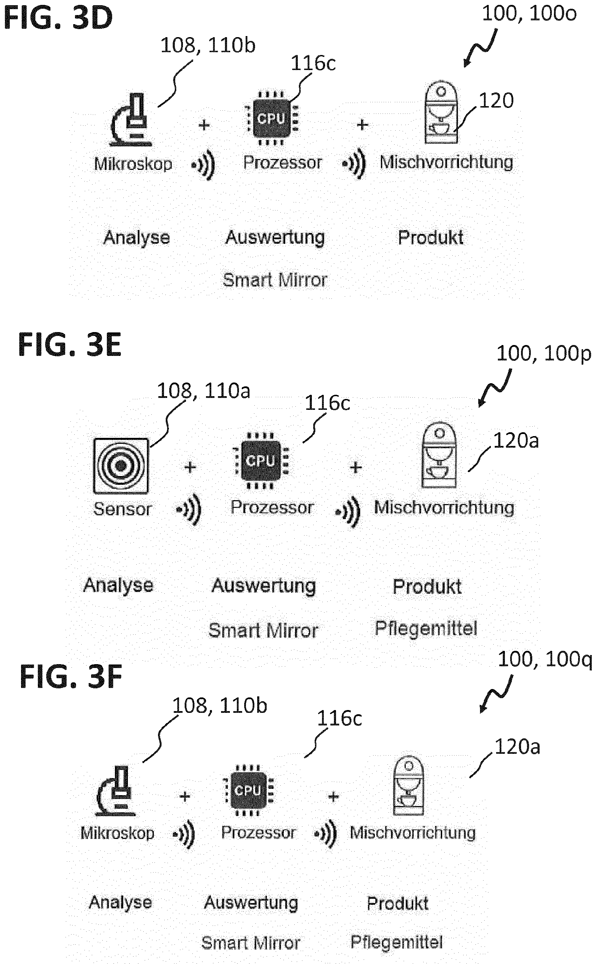

Die Vorrichtung 100o aus FIG. 3D weist ein smartes Endgerät mit Mikroskop(aufsatz) 110b, eine Datenverarbeitungsvorrichtung 116c als Teil eines Smart Mirror 110c und eine generische Mischvorrichtung 120 auf. Daten können von dem Sensor 110a an den Smart Mirror 116c als Signal 114 übermittelt werden, und der Smart Mirror 116c kann der Mischvorrichtung 120 die Zusammensetzung des Haarbehandlungsmittels als ein Signal 118 übermitteln.

Die Vorrichtung 100p aus FIG. 3E weist einen generischen Sensor 110a, eine Datenverarbeitungsvorrichtung 116c als Teil eines Smart Mirror 110c und eine Mischvorrichtung 120a zum Erzeugen eines Pflegemittels auf. Der generische Sensor 110a kann beispielsweise eine Bürste mit mindestens einem Sensor 110a sein. Daten können von dem Sensor 110a an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln.

Die Vorrichtung 100q aus FIG. 3F weist ein smartes Endgerät mit Mikroskop(aufsatz) 110b, eine Datenverarbeitungsvorrichtung 116c als Teil eines Smart Mirror 110c und eine Mischvorrichtung 120a zum Erzeugen eines Pflegemittels auf. Daten können von dem Sensor 110a an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln.

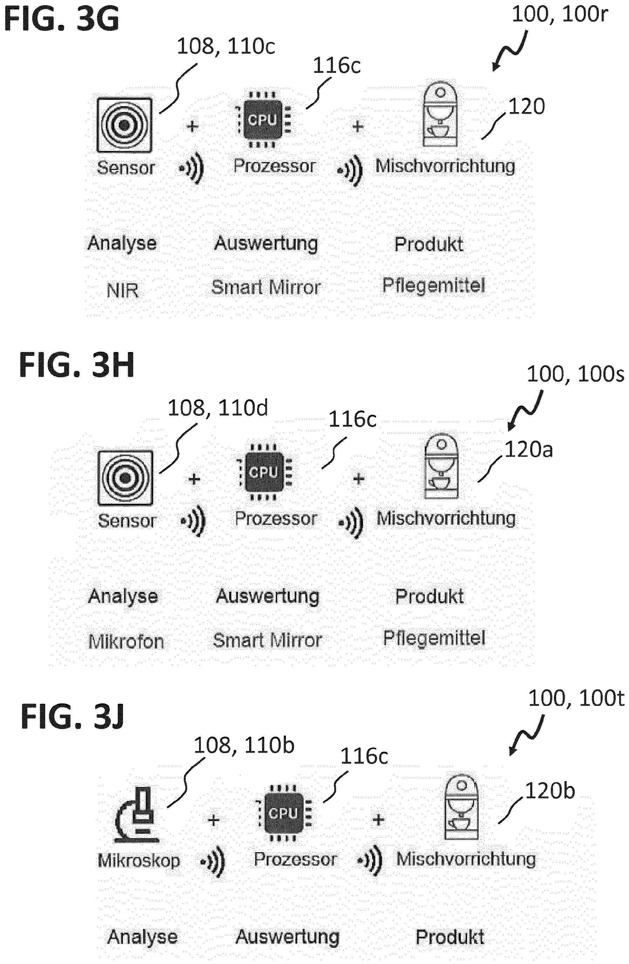

Die Vorrichtung 100r aus FIG. 3G weist einen Sensor 110c für Nahinfrarotlicht zum Ermitteln der Haarschädigung, z.B. anhand eines Cysteinsäuregehalts, und/oder zum Ermitteln eines Haarstatus auf, eine Datenverarbeitungsvorrichtung 116c als Teil eines Smart Mirror 110c und eine Mischvorrichtung 120a zum Erzeugen eines Pflegemittels auf. Daten können von dem Sensor 110c an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln. Alternativ, nicht dargestellt, kann anstelle des NIR-Sensors 110c, ein Sensor zum Erfassen von Fluoreszenzlicht eingesetzt werden.

Die Vorrichtung 100s aus FIG. 3H weist ein Mikrofon 110d zum Ermitteln der Oberflächenrauhigkeit), eine Datenverarbeitungsvorrichtung 116c als Teil eines Smart Mirror 110c und eine Mischvorrichtung 120a zum Erzeugen eines Pflegemittels auf. Daten können von dem Sensor 110c an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln.

Die Vorrichtung 100t aus FIG. 3J weist ein smartes Endgerät mit Mikroskop(aufsatz) 110b zum Ermitteln der Haarschädigung (z.B. Dehnungsschäden), eine Datenverarbeitungsvorrichtung 116c als Teil eines Smart Mirror 110c und eine Mischvorrichtung 120b zum Erzeugen eines Haarfärbemittels auf. Daten können von dem Sensor 110a an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln.

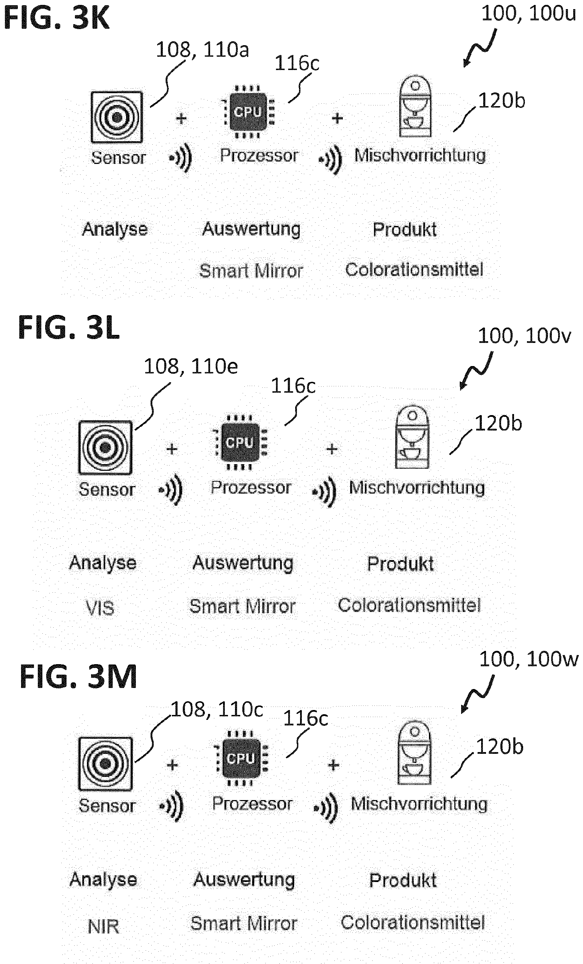

Die Vorrichtung 100u aus FIG. 3K weist einen generischen Sensor 110a, eine Datenverarbeitungsvorrichtung 116c als Teil eines Smart Mirror 110c und eine Mischvorrichtung 120b zum Erzeugen eines Haarfärbemittels auf. Der generische Sensor 110a kann beispielsweise eine Bürste mit mindestens einem Sensor 110a sein. Daten können von dem Sensor 110a an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln.

Die Vorrichtung 100v aus FIG. 3L weist einen Sensor 110e für sichtbares Licht zum Ermitteln der Haarfarbe, z.B. eine digitale Farbkamera oder einen VIS-Sensor, eine Datenverarbeitungsvorrichtung 116c als Teil eines Smart Mirror 110c und eine Mischvorrichtung 120b zum Erzeugen eines Haarfärbemittels auf. Daten können von dem Sensor 110e an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln.

Die Vorrichtung 100w aus FIG. 3M weist einen Sensor 110c für Nahinfrarotlicht zum Ermitteln der Haarschädigung, z.B. anhand eines Cysteinsäuregehalts, und/oder zum Ermitteln eines Haarstatus auf, eine Datenverarbeitungsvorrichtung 116c als Teil eines Smart Mirror 110c und eine Mischvorrichtung 120b zum Erzeugen eines Haarfärbemittels auf. Daten können von dem Sensor 110c an den Smart Mirror 116c als Signal 114 übermittelt werden, und der Smart Mirror 116c kann der Mischvorrichtung 120a die Zusammensetzung des Haarbehandlungsmittels als ein Signal 118 übermitteln. Alternativ, nicht dargestellt, kann anstelle des NIR-Sensors 110c, ein Sensor zum Erfassen von Fluoreszenzlicht eingesetzt werden. In einer weiteren, nicht dargestellten, Alternative kann anstelle des NIR-Sensors 110c, ein Sensor zum Erfassen von sichtbarem Licht und (N)IR-Licht eingesetzt werden.

Die Vorrichtung 100x aus FIG. 4A weist einen generischen Sensor 110a und, als integrierte Vorrichtung 118, eine Datenverarbeitungsvorrichtung 116d und eine generische Mischvorrichtung 120 auf. Der generische Sensor 110a kann beispielsweise eine Bürste mit mindestens einem Sensor 110a sein. Die integrierte Vorrichtung 118 kann eine Mischvorrichtung 120 mit einem Prozessor und einem optischen und/oder akustischen Ein- und/oder Ausgabegerät sein.

Die Vorrichtung 100y aus FIG. 4B weist ein smartes Endgerät mit Mikroskop(aufsatz) 110b und, als integrierte Vorrichtung 118, eine Datenverarbeitungsvorrichtung 116d und eine generische Mischvorrichtung 120 auf. Die integrierte Vorrichtung 118 kann eine Mischvorrichtung 120 mit einem Prozessor und einem optischen und/oder akustischen Ein- und/oder Ausgabegerät sein.

Die Vorrichtung 100za aus FIG. 4C weist einen generischen Sensor 110a und, als integrierte Vorrichtung 118, eine Datenverarbeitungsvorrichtung 116d und eine Mischvorrichtung 120a zum Erzeugen eines Pflegemittels auf. Der generische Sensor 110a kann beispielsweise eine Bürste mit mindestens einem Sensor 110a sein. Die integrierte Vorrichtung 118 kann eine Mischvorrichtung 120 mit einem Prozessor und einem optischen und/oder akustischen Ein- und/oder Ausgabegerät sein.

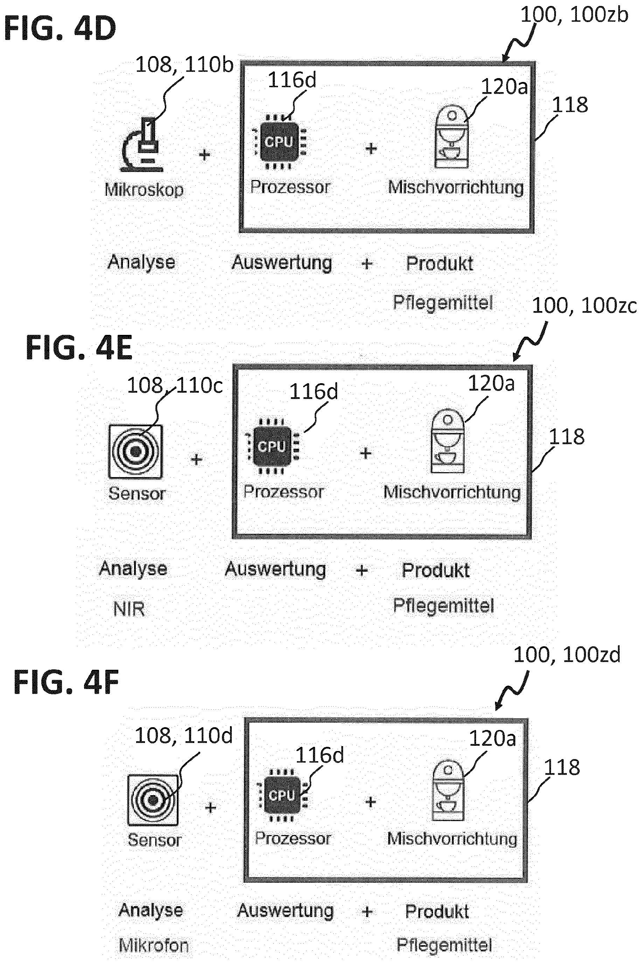

Die Vorrichtung 100zb aus FIG. 4D weist ein smartes Endgerät mit Mikroskop(aufsatz) 110b und, als integrierte Vorrichtung 118, eine Datenverarbeitungsvorrichtung 116d und eine Mischvorrichtung 120a zum Erzeugen eines Pflegemittels auf. Die integrierte Vorrichtung 118 kann eine Mischvorrichtung 120 mit einem Prozessor und einem optischen und/oder akustischen Ein- und/oder Ausgabegerät sein.

Die Vorrichtung 100zc aus FIG. 4E weist einen Sensor 110c für Nahinfrarotlicht zum Ermitteln der Haarschädigung, z.B. anhand eines Cysteinsäuregehalts, und/oder zum Ermitteln des Haarstatus auf, und, als integrierte Vorrichtung 118, eine Datenverarbeitungsvorrichtung 116d und eine Mischvorrichtung 120a zum Erzeugen eines Pflegemittels auf. Die integrierte Vorrichtung 118 kann eine Mischvorrichtung 120 mit einem Prozessor und einem optischen und/oder akustischen Ein- und/oder Ausgabegerät sein. Alternativ, nicht dargestellt, kann anstelle des NIR-Sensors 110c, ein Sensor zum Erfassen von Fluoreszenzlicht eingesetzt werden.

Die Vorrichtung 100zd aus FIG. 4F weist ein Mikrofon 110d zum Ermitteln der Oberflächenrauhigkeit), und, als integrierte Vorrichtung 118, eine Datenverarbeitungsvorrichtung 116d und eine Mischvorrichtung 120a zum Erzeugen eines Pflegemittels auf. Die integrierte Vorrichtung 118 kann eine Mischvorrichtung 120 mit einem Prozessor und einem optischen und/oder akustischen Ein- und/oder Ausgabegerät sein.

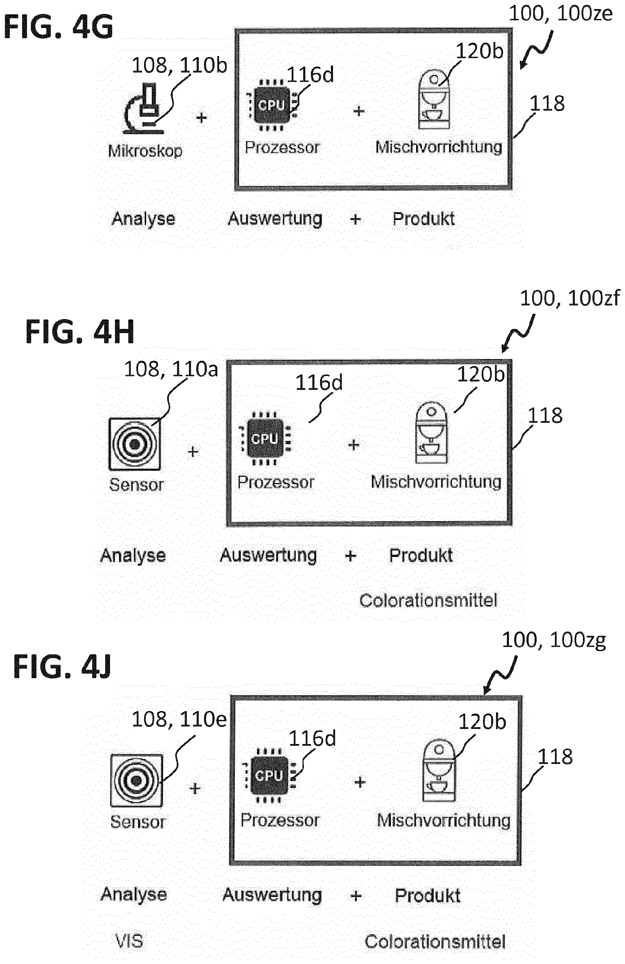

Die Vorrichtung 100ze aus FIG. 4G weist ein smartes Endgerät mit Mikroskop(aufsatz) 110b und, als integrierte Vorrichtung 118, eine Datenverarbeitungsvorrichtung 116d und eine Mischvorrichtung 120b zum Erzeugen eines Haarfärbemittels auf. Die integrierte Vorrichtung 118 kann eine Mischvorrichtung 120 mit einem Prozessor und einem optischen und/oder akustischen Ein- und/oder Ausgabegerät sein.

Die Vorrichtung 100zg aus FIG. 4H weist einen generischen Sensor 110a und, als integrierte Vorrichtung 118, eine Datenverarbeitungsvorrichtung 116d und eine Mischvorrichtung 120b zum Erzeugen eines Haarfärbemittels auf. Der generische Sensor 110a kann beispielsweise eine Bürste mit mindestens einem Sensor 110a sein. Die integrierte Vorrichtung 118 kann eine Mischvorrichtung 120 mit einem Prozessor und einem optischen und/oder akustischen Ein- und/oder Ausgabegerät sein.

Die Vorrichtung 100zg aus FIG. 4J weist einen Sensor 110e für sichtbares Licht zum Ermitteln der Haarfarbe, z.B. eine digitale Farbkamera oder einen VIS-Sensor, und, als integrierte Vorrichtung 118, eine Datenverarbeitungsvorrichtung 116d und eine Mischvorrichtung 120b zum Erzeugen eines Haarfärbemittels auf. Die integrierte Vorrichtung 118 kann eine Mischvorrichtung 120 mit einem Prozessor und einem optischen und/oder akustischen Ein- und/oder Ausgabegerät sein.

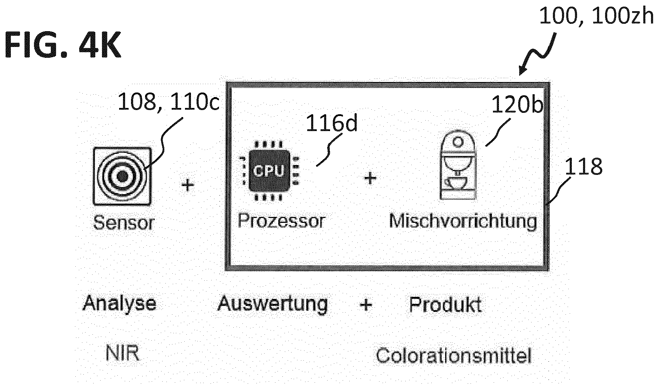

Die Vorrichtung 100zh aus FIG. 4K weist einen Sensor 110c für Nahinfrarotlicht zum Ermitteln der Haarschädigung, z.B. anhand eines Cysteinsäuregehalts, und/oder zum Ermitteln eines Haarstatus auf, und, als integrierte Vorrichtung 118, eine Datenverarbeitungsvorrichtung 116d und eine Mischvorrichtung 120a zum Erzeugen eines Haarfärbemittels auf. Die integrierte Vorrichtung 118 kann eine Mischvorrichtung 120 mit einem Prozessor und einem optischen und/oder akustischen Ein- und/oder Ausgabegerät sein. Alternativ, nicht dargestellt, kann anstelle des NIR-Sensors 110c, ein Sensor zum Erfassen von Fluoreszenzlicht eingesetzt werden. In einer weiteren, nicht dargestellten, Alternative kann anstelle des NIR-Sensors 110c, ein Sensor zum Erfassen von sichtbarem Licht und (N)IR-Licht eingesetzt werden.

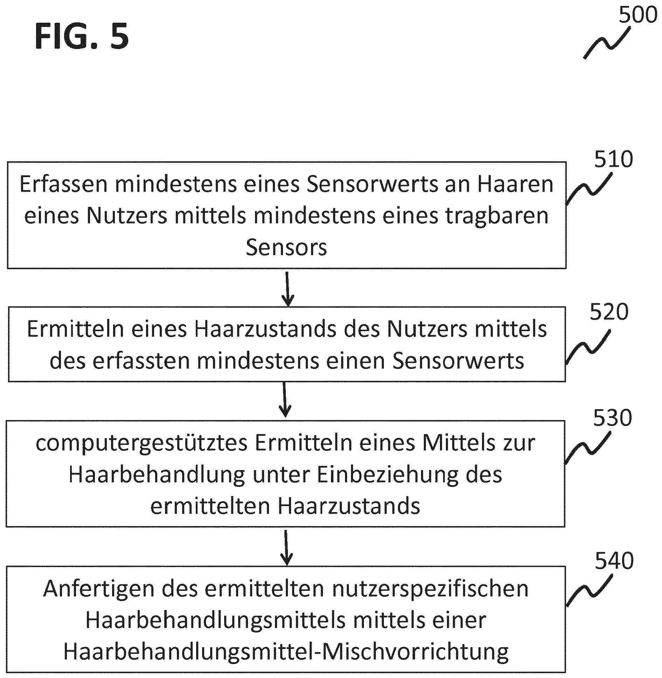

FIG. 5 zeigt ein Ablaufdiagramm 500, welches ein Verfahren zum Bereitstellen eines Haarbehandlungsmittels gemäß verschiedenen Ausführungsbeispielen darstellt. Zum Ausführen des Verfahrens kann eine Vorrichtung gemäß verschiedenen Ausführungsbeispielen wie oben beschrieben verwendet werden.

Das Verfahren kann aufweisen ein Erfassen mindestens eines Sensorwerts an Haaren eines Nutzers mittels mindestens eines tragbaren Sensors (bei 510), ein Ermitteln eines Haarzustands des Nutzers mittels des erfassten mindestens einen Sensorwerts (bei 520), ein computergestütztes Ermitteln eines Mittels zur Haarbehandlung unter Einbeziehung des ermittelten Haarzustands (bei 530) und ein Anfertigen des ermittelten nutzerspezifischen Haarbehandlungsmittels mittels einer Haarbehandlungsmittel-Mischvorrichtung (bei 540).

In verschiedenen Ausführungsbeispielen kann das Ermitteln des Haarzustands zumindest ein Ermitteln eines Schädigungsgrad des Haars aufweisen.

In verschiedenen Ausführungsbeispielen kann das Ermitteln des Haarzustands zumindest ein Ermitteln eines Haarstatus aufweisen.

Weitere vorteilhafte Ausgestaltungen des Verfahrens ergeben sich aus der Beschreibung der Vorrichtung und umgekehrt.

## Patentansprüche

1. Vorrichtung (100, 100f, 100j, 100k, 100r, 100v, 100w, 100zc, 100zg, 100zh) zum Bereitstellen eines Haarbehandlungsmittels, aufweisend:
eine tragbare Sensorvorrichtung (108) mit mindestens einem Sensor (110, 110a) zum Erfassen mindestens eines Sensorwerts an Haaren eines Nutzers;
eine Datenverarbeitungsvorrichtung (116, 116b, 116c, 116d) zum Ermitteln eines Haarzustands des Nutzers mittels des erfassten mindestens einen Sensorwerts, und zum computergestützten Ermitteln eines nutzerspezifischen Haarbehandlungsmittels unter Einbeziehung des ermittelten Haarzustands; und
eine Haarbehandlungsmittel-Mischvorrichtung (120, 120a, 120b) zum Anfertigen des ermittelten nutzerspezifischen Haarbehandlungsmittels,
wobei die Vorrichtung **dadurch gekennzeichnet ist, dass** der mindestens eine Sensor mindestens einen optischen Sensor (110c, 110e) zum Ermitteln eines Gehalts eines Haarinhaltsstoffes des Haars des Nutzers aufweist, wobei der Gehalt eines Haarinhaltsstoffes wenigstens ein Cysteinsäuregehalt ist,
und das Ermitteln des Haarzustands zumindest ein Ermitteln eines Schädigungsgrads des Haars aufweist.

2. Vorrichtung gemäß Anspruch 1,
wobei der mindestens eine Sensor ein Mikrofon (110d) zum Ermitteln einer Oberflächenrauhigkeit des Haars aufweist.

3. Vorrichtung gemäß einem der vorhergehenden Ansprüche,
wobei die Datenverarbeitungsvorrichtung Teil eines Smartphones, Tablets oder eines Smart Mirrors (110c) ist.

4. Vorrichtung gemäß einem der vorhergehenden Ansprüche,
wobei die Haarbehandlungsmittel-Mischvorrichtung eine Mischvorrichtung zum Erzeugen eines Haarpflegemittels, eines Haarfärbemittels und/oder eines Haarstylingmittels aufweist.

5. Vorrichtung gemäß einem der vorhergehenden Ansprüche,
wobei die Sensorvorrichtung und die Datenverarbeitungsvorrichtung eine integrierte tragbare Vorrichtung (112) bilden.

6. Vorrichtung gemäß einem der vorhergehenden Ansprüche,
wobei die Datenverarbeitungsvorrichtung und die Haarbehandlungsmittel-Mischvorrichtung eine integrierte Vorrichtung (118) bilden.

7. Verfahren zum Bereitstellen eines Haarbehandlungsmittels mittels einer Vorrichtung
gemäß einem der vorhergehenden Ansprüche, aufweisend:
Erfassen (510) mindestens eines Sensorwerts an Haaren eines Nutzers mittels einer Sensorvorrichtung, die mindestens einen tragbaren Sensor aufweist;
Ermitteln (520) eines Haarzustands des Nutzers mittels des erfassten mindestens einen Sensorwerts, wobei das Ermitteln des Haarzustands zumindest ein Ermitteln eines Schädigungsgrads des Haars und/oder Haarstatus aufweist,
computergestütztes Ermitteln (530) eines nutzerspezifischen Haarbehandlungsmittels unter Einbeziehung des ermittelten Haarzustands; und
Anfertigen (540) des ermittelten nutzerspezifischen Haarbehandlungsmittels mittels einer Haarbehandlungsmittel-Mischvorrichtung,
wobei das Verfahren **dadurch gekennzeichnet ist, dass** das Ermitteln des Schädigungsgrads des Haars ein Ermitteln eines Cysteinsäuregehalt des Haars aufweist.

8. Verfahren gemäß Anspruch 7,
wobei das Ermitteln des Schädigungsgrads des Haars ein Ermitteln einer Oberflächenrauhigkeit des Haars aufweist.

9. Verfahren gemäß einem der Ansprüche 7 oder 8, ferner aufweisend:
Bereitstellen eines Behandlungsziels durch den Nutzer.

10. Verfahren gemäß einem der Ansprüche 7 bis 9,
wobei das Behandlungsmittel ein Haarfärbemittel, ein Haarpflegemittel und/oder ein Haarumstylingmittel aufweist.

11. Verfahren gemäß einem der Ansprüche 7 bis 10, ferner aufweisend:
Übermitteln des erfassten Sensorwerts von dem Sensor an eine Datenverarbeitungsvorrichtung und/oder Übermitteln des ermittelten Haarbehandlungsmittels von der Datenverarbeitungsvorrichtung an die Haarbehandlungsmittel-Mischvorrichtung.

12. Verfahren gemäß Anspruch 11, **dadurch gekennzeichnet, dass**
die Datenverarbeitungsvorrichtung ferner eingerichtet ist, den erfassten Sensorwert und/oder teilweise durch die Datenverarbeitungsvorrichtung ausgewertete Sensordaten und/oder einen ermittelten Haarzustand an eine externe Datenverarbeitungsvorrichtung, beispielsweise an eine Cloud-Serverarchitektur, zu übermitteln und ein Ergebnis von der externen Datenverarbeitungsvorrichtung zu empfangen.

## Claims

1. A device (100, 100f, 100j, 100k, 100r, 100v, 100w, 100zc, 100zg, 100zh) for preparing a hair-treatment agent, comprising:
a portable sensor device (108) having at least one sensor (110, 110a) for detecting at least one sensor value on hairs of a user;
a data-processing device (116, 116b, 116c, 116d) for determining a hair state of the user by means of the detected at least one sensor value, and for computer-aided determination of a user-specific hair-treatment agent taking into account the determined hair state; and
a hair-treatment-agent mixing device (120, 120a, 120b) for producing the determined user-specific hair-treatment agent,
the device being **characterized in that** the at least one sensor comprises at least one optical sensor (110c, 110e) for determining a content of a hair constituent of the hair of the user, the content of a hair constituent being at least one cysteic-acid content,
and the determination of the hair state comprises at least determining a degree of damage to the hair.

2. The device according to claim 1,
wherein the at least one sensor comprises a microphone (110d) for determining a surface roughness of the hair.

3. The device according to one of the preceding claims,
wherein the data-processing device is part of a smartphone, tablet, or a smart mirror (110c).

4. The device according to one of the preceding claims,
wherein the hair-treatment-agent mixing device comprises a mixing device for producing a hair-care agent, a hair dye and/or a hair-styling agent.

5. The device according to one of the preceding claims,
wherein the sensor device and the data-processing device form an integrated portable device (112).

6. The device according to one of the preceding claims,
wherein the data-processing device and the hair-treatment-agent mixing device form an integrated device (118).

7. A method for preparing a hair-treatment agent by means of a device according to one of the preceding claims, the method comprising:
detecting (510) at least one sensor value on hairs of a user by means of a sensor device which has at least one portable sensor;
determining (520) a hair state of the user by means of the detected at least one sensor value, the determination of the hair state comprising at least a determination of a degree of damage to the hair and/or hair status,
computer-aided determination (530) of a user-specific hair-treatment agent taking into account the determined hair state; and
producing (540) the determined user-specific hair-treatment agent by means of a hair-treatment-agent mixing device,
the method being **characterized in that** the determination of the degree of damage to the hair comprises determining a cysteic-acid content of the hair.

8. The method according to claim 7,
wherein the determination of the degree of damage to the hair comprises determining a surface roughness of the hair.

9. The method according to one of claims 7 or 8, further comprising:
provision of a treatment target by the user.

10. The method according to one of claims 7 to 9,
wherein the treatment agent comprises a hair dye, a hair-care agent and/or a hair-styling agent.

11. The method according to one of claims 7 to 10, further comprising:
transmitting the detected sensor value from the sensor to a data-processing device and/or
transmitting the determined hair-treatment agent from the data-processing device to the hair-treatment-agent mixing device.

12. The method according to claim 11, **characterized in that**
the data-processing device is further configured to transmit the detected sensor value and/or sensor data evaluated partially by the data-processing device and/or a determined hair state to an external data-processing device, for example to a cloud server architecture, and to receive a result from the external data-processing device.

## Revendications

1. Dispositif (100, 100f, 100j, 100k, 100r, 100v, 100w, 100zc, 100zg, 100zh) permettant la mise à disposition d'un agent de traitement capillaire, présentant :
un dispositif de capteur portable (108) comportant au moins un capteur (110, 110a) pour la détection d'au moins une valeur de capteur sur des cheveux d'un utilisateur ;
un dispositif de traitement de données (116, 116b, 116c, 116d) pour la détermination d'un état capillaire de l'utilisateur au moyen de l'au moins une valeur de capteur détectée, et pour la détermination assistée par ordinateur d'un agent de traitement capillaire spécifique à l'utilisateur en tenant compte de l'état capillaire déterminé ; et
un dispositif de mélange d'agent de traitement capillaire (120, 120a, 120b) pour la préparation de l'agent de traitement capillaire spécifique à l'utilisateur déterminé,
dans lequel le dispositif est **caractérisé en ce que** l'au moins un capteur présente au moins un capteur optique (110c, 110e) pour la détermination d'une teneur en composant capillaire des cheveux de l'utilisateur, la teneur en composant capillaire étant au moins une teneur en acide cystéique,
et la détermination de l'état capillaire présente au moins une détermination d'un degré d'endommagement des cheveux.

2. Dispositif selon la revendication 1,
dans lequel l'au moins un capteur présente un microphone (110d) pour la détermination d'une rugosité de surface des cheveux.

3. Dispositif selon l'une des revendications précédentes,
dans lequel le dispositif de traitement de données fait partie d'un téléphone intelligent, d'une tablette ou d'un miroir intelligent (110c).

4. Dispositif selon l'une des revendications précédentes,
dans lequel le dispositif de mélange d'agent de traitement capillaire présente un dispositif de mélange pour la production d'un agent de soin capillaire, d'un agent de coloration capillaire et/ou d'un agent de coiffage.

5. Dispositif selon l'une des revendications précédentes,
dans lequel le dispositif de capteur et le dispositif de traitement de données forment un dispositif portable intégré (112).

6. Dispositif selon l'une des revendications précédentes,
dans lequel le dispositif de traitement de données et le dispositif de mélange d'agent de traitement capillaire forment un dispositif intégré (118).

7. Procédé permettant la mise à disposition d'un agent de traitement capillaire au moyen d'un dispositif selon l'une des revendications précédentes, présentant :
la détection (510) d'au moins une valeur de capteur sur des cheveux d'un utilisateur au moyen d'un dispositif de capteur qui présente au moins un capteur portable ;
la détermination (520) d'un état capillaire de l'utilisateur au moyen de l'au moins une valeur de capteur détectée, la détermination de l'état capillaire présentant au moins une détermination d'un degré d'endommagement des cheveux et/ou de la structure capillaire,
la détermination assistée par ordinateur (530) d'un agent de traitement capillaire spécifique à l'utilisateur en tenant compte de l'état capillaire déterminé ; et
la préparation (540) de l'agent de traitement capillaire spécifique à l'utilisateur déterminé au moyen d'un dispositif de mélange d'agent de traitement capillaire,
le procédé étant **caractérisé en ce que** la détermination du degré d'endommagement des cheveux présente une détermination d'une teneur en acide cystéique des cheveux.

8. Procédé selon la revendication 7,
dans lequel la détermination du degré d'endommagement des cheveux présente une détermination d'une rugosité de surface des cheveux.

9. Procédé selon l'une des revendications 7 ou 8, présentant en outre :
la mise à disposition d'un objectif de traitement par l'utilisateur.

10. Procédé selon l'une des revendications 7 à 9,
dans lequel l'agent de traitement présente un agent de coloration capillaire, un agent de soin capillaire et/ou un agent de coiffage.

11. Procédé selon l'une des revendications 7 à 10, présentant en outre :
la transmission de la valeur de capteur détectée depuis le capteur à un dispositif de traitement de données et/ou la transmission de l'agent de traitement capillaire déterminé depuis le dispositif de traitement de données au dispositif de mélange d'agent de traitement capillaire.

12. Procédé selon la revendication 11, **caractérisé en ce que**
le dispositif de traitement de données est en outre configuré pour transmettre la valeur de capteur détectée et/ou des données de capteur partiellement évaluées par le dispositif de traitement de données et/ou un état capillaire déterminé à un dispositif de traitement de données externe, par exemple à une architecture de serveur infonuagique, et pour recevoir un résultat en provenance du dispositif de traitement de données externe.
